# EUROPEAN PATENT APPLICATION

(11) **EP 2 633 880 A1**
(43) Date of publication of application: **04.09.2013**
(21) Application number: 11835898.5
(22) Date of filing: 26.05.2011
(51) Int. Cl.: A61M 37/00

(54) **METHOD FOR OPERATING A DEVICE FOR DELIVERING A SUBSTANCE TO BE INTRODUCED, AND METHOD FOR DELIVERING A SUBSTANCE TO BE INTRODUCED**

(30) Priority: 25.10.2010 JP 2010238885
(71) Applicant: The Ritsumeikan Trust, Kyoto-shi, Kyoto 604-8520 (JP); Kyoto University, Sakyo-ku Kyoto-shi Kyoto 606-8501 (JP)
(72) Inventor: SHIMIZU, Kazunori, Kyoto-shi Kyoto 606-8501 (JP); KONISHI, Satoshi, Kusatsu-shi Shiga 525-8577 (JP); KAWAKAMI, Shigeru, Kyoto-shi Kyoto 606-8501 (JP); HASHIDA, Mitsuru, Kyoto-shi Kyoto 606-8501 (JP)
(74) Representative: De Clercq, Ann G. Y.
(86) International application number: PCT/JP2011/062102
(87) International publication number: WO 2012/056756

(57) **Abstract**

Provided are a method for delivering a substance to be introduced and a method for operating a device for delivering a substance to be introduced, in which, in a subject, by supplying the substance to be introduced to a site different from a target site and thereafter applying pressure to the target site in the subject, the substance to be introduced is delivered into the target site.

## Description

### TECHNICAL FIELD

The present invention relates to a method for operating a device for delivering a substance to be introduced, and a method for delivering a substance to be introduced.

### BACKGROUND ART

In therapy, diagnosis and the like for diseases, a variety of substances which target a molecule in a living organism are used, the substances including a pharmaceutical, a protein, a peptide, an antibody, a nucleic acid, and the like. For example, a drug containing a nucleic acid (hereinafter referred to as "nucleic acid drug") has an ability to control transcription of DNA to mRNA, translation of mRNA to a protein, physiological activity of a protein, and the like. Therefore, the nucleic acid drug is expected to be applied to therapy for various diseases. In order to allow the nucleic acid drug to exhibit a sufficient effect, it is desirable to efficiently deliver the nucleic acid drug into a target site.

As a method for delivering a nucleic acid into a target site in an organ or the like, there has been proposed, for example, a method for injecting directly a nucleic acid into the target site by a syringe (hereinafter referred to as the "direct injection method"), a method for injecting a nucleic acid into a region isolated by placement of a catheter into a vascular structure of an organ to occlude the flow of body fluid (see, for example, Patent Literature 1, Non Patent literatures 1 and 2, and the like), and the like.

### CITATION LIST

### {PATENT LITERATURE}

{Patent Literature 1} Japanese Unexamined Patent Application Publication No. 2006-501177

### {NON PATENT LITERATURE}

{Non Patent Literature 1} Simon J. Eastman et al, "Development of catheter-based procedures for transducing the isolated rabbit liver with plasmid DNA," Human Gene Therapy, November 20, 2002, Vol. 13, pp. 2065-2077
{Non Patent Literature 2} SE Khorsandi et al, "Minimally invasive and selective hydrodynamic gene therapy of liver segments in the pig and human," Cancer Gene Therapy, 2008, Vol. 15, pp. 225-230

### SUMMARY OF THE INVENTION

### PROBLEMS TO BE SOLVED BY THE INVENTION

However, the above direct injection method has a drawback such that a needle of the syringe may cause damage to tissue around the target site.
In addition, the methods described in Patent Literature 1 and Non Patent Literatures 1 and 2 have a drawback such that although a nucleic acid can be delivered into a region isolated by the catheter, it is difficult to locally deliver the nucleic acid into only single target site in the organ or the like, or to deliver the nucleic acid into a plurality of target sites simultaneously.

The present invention has been accomplished in view of the above-mentioned prior arts. An object of the present invention is to provide a method for operating a device for delivering a substance to be introduced and a method for delivering a substance to be introduced, that can efficiently locally deliver a substance to be introduced into a target site.

### SOLUTION TO THE PROBLEMS

The gist of the present invention relates to:
(1) a method for operating a device for delivering a substance to be introduced, the device including
   a supply unit configured to supply the substance to be introduced to a vascular path located upstream of a target site in a tissue existed in a living organism, and
   a pressure applying unit configured to apply pressure to the target site in the tissue,
   the method including the steps of:
   (1-1) causing the supply unit to discharge the substance to be introduced, thereby supplying the substance to be introduced to the vascular path, and
   (1-2) simultaneously with or after the step (1-1), causing the pressure applying unit to generate pressure against the target site in the tissue;
(2) the method according to the above item (1), wherein the substance to be introduced is a nucleic acid or a peptide compound;
(3) the method according to the above item (1) or (2), wherein the pressure applying unit includes a suction part configured to apply suction at the target site, and
   wherein in the step (1-2), suction pressure against the target site is generated by causing the suction part to apply suction at the target site;
(4) the method according to the above item (1) or (2), wherein the pressure applying unit includes a pressing unit configured to press the target site, and a holding unit configured to positionally-fix and hold the tissue, and
   wherein in the step (1-2), a pressing force against the target site is generated by causing the pressing unit to press the target site;
(5) the method according to the above item (4), wherein the holding unit is implantable in the living organism;
(6) a method for delivering a substance to be introduced into a target site in a tissue existed in a non-human mammal, the method including the steps of:
   (2-1) supplying the substance to be introduced to a vascular path located upstream of the tissue; and
   (2-2) simultaneously with or after the step (2-1), applying pressure to the target site in the tissue, thereby delivering the substance to be introduced into the target site;
(7) the method according to the above item (6), wherein the substance to be introduced is a nucleic acid;
(8) the method according to (6) or (7), wherein in the step (2-2), suction pressure is applied to the target site;
(9) the method according to the item (6) or (7), wherein in the step (2-2), the target site is pressed while positionally-fixing the tissue;
(10) the method according to the item (9), wherein the tissue is an organ tissue and
   wherein in the step (2-2), the target site is pressed while the tissue is positionally-fixed, the organ being accommodated in a case capable of accommodating the organ;
(11) a method for delivering a substance to be introduced into a target site in a subject derived from a living organism, the method including the steps of:
   (3-1) supplying the substance to be introduced to a site different from the target site in the subject; and
   (3-2) simultaneously with or after the step (3-1), applying pressure to the target site in the subject, thereby delivering the substance to be introduced into the target site.
(12) the method according to the above item (11), wherein the substance to be introduced is a nucleic acid or a peptide compound;
(13) the method according to the above item (11) or (12), wherein in the step (3-2), suction pressure is applied to the target site; and
(14) the method according to the above item (11) or (12), wherein in the step (3-2), the target site is pressed while positionally-fixing the target site.

### ADVANTAGEOUS EFFECTS OF THE INVENTION

According to the method for operating a device for delivering a substance to be introduced of the present invention and the method for delivering a substance to be introduced of the present invention, the substance to be introduced can be efficiently locally delivered into a target site.

### BRIEF DESCRIPTION OF THE DRAWINGS

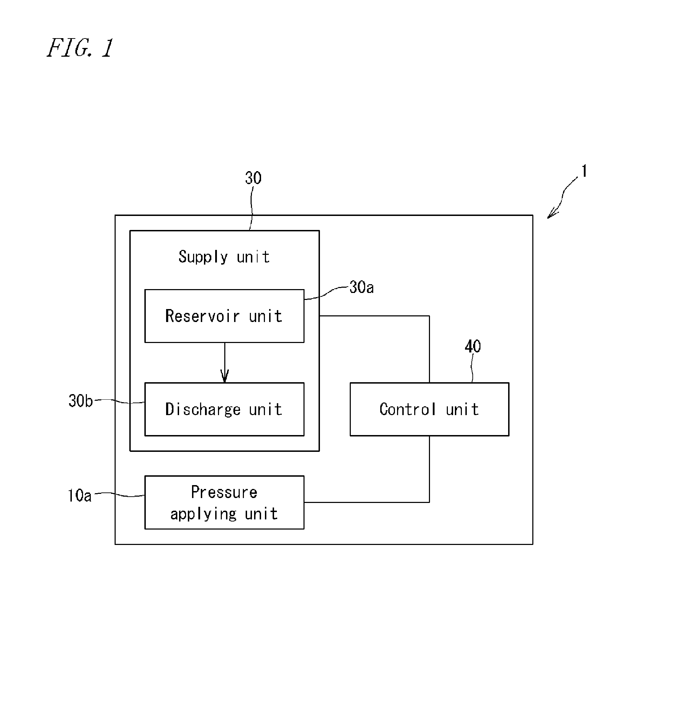

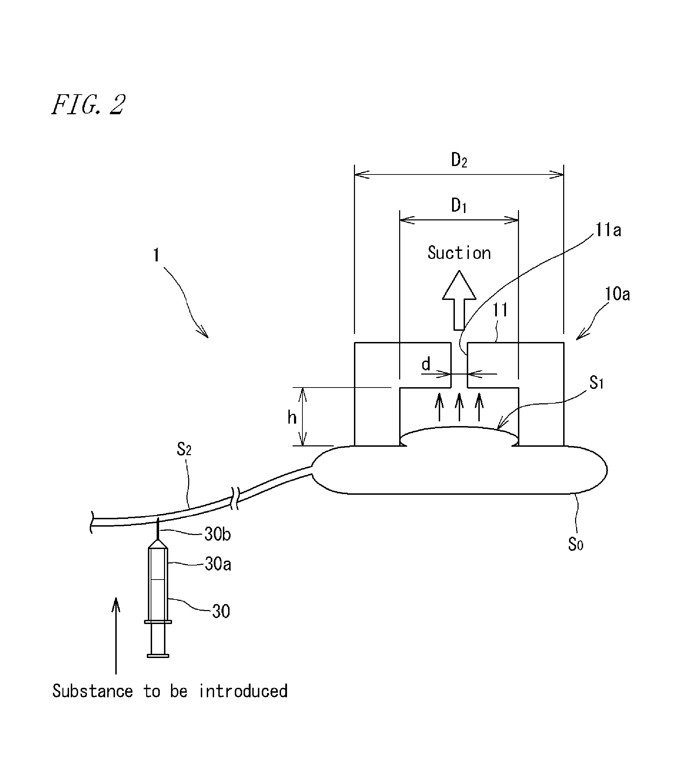

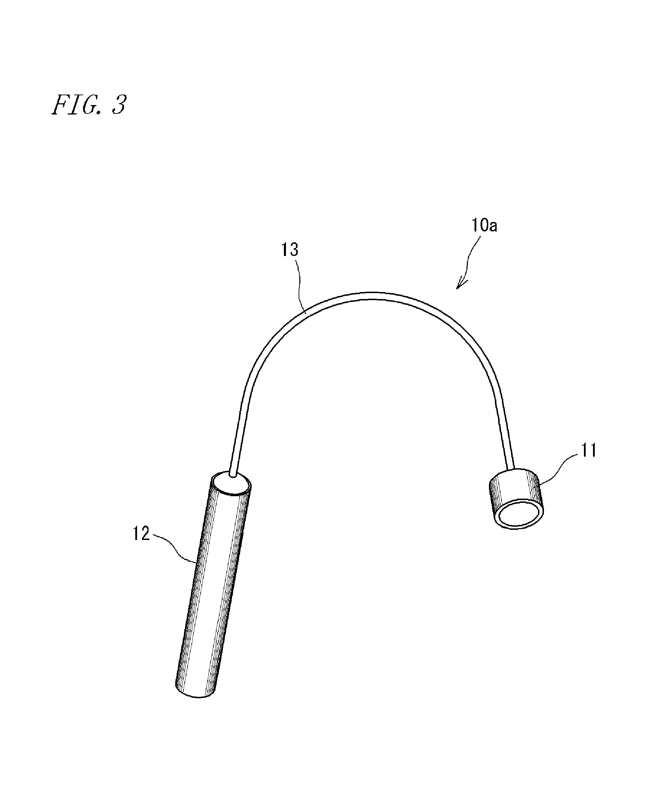

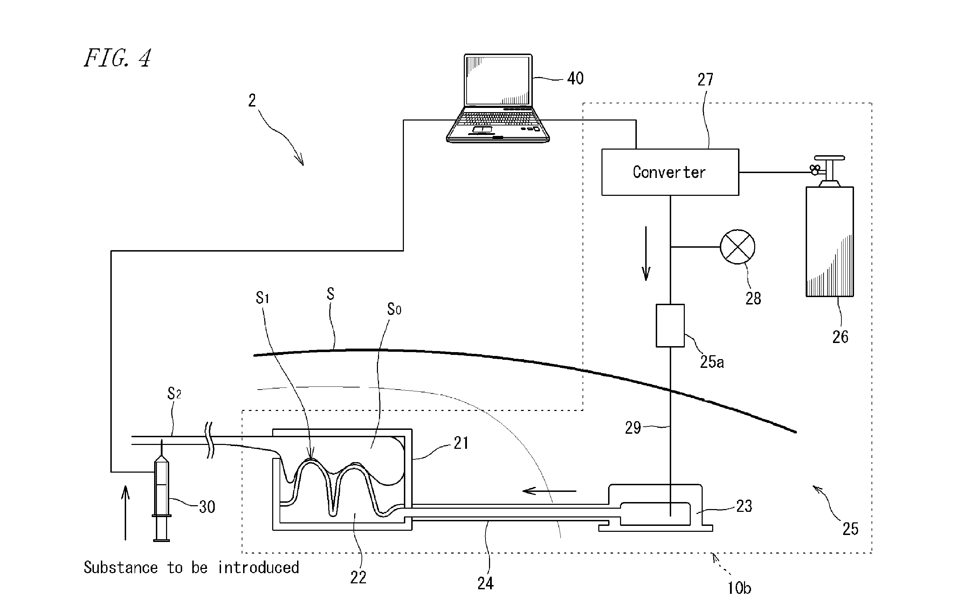

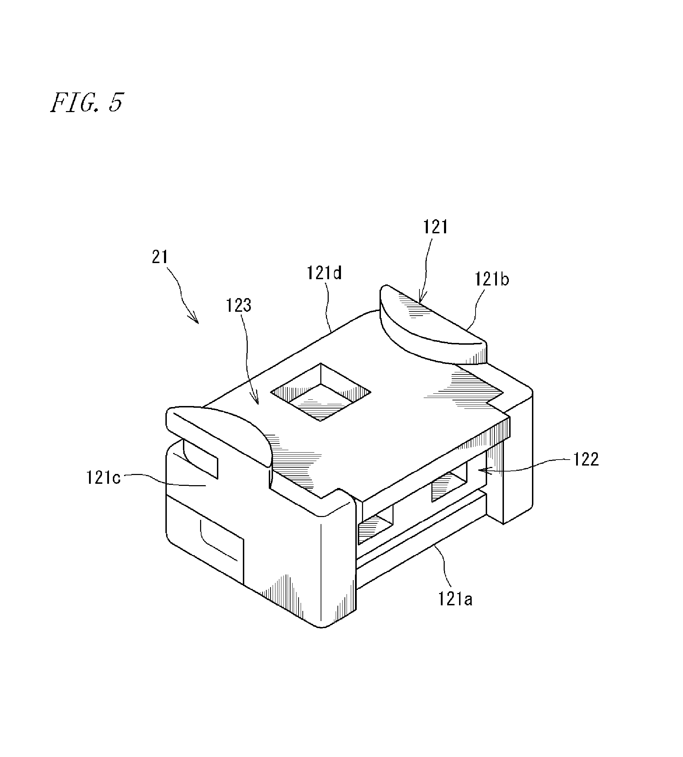

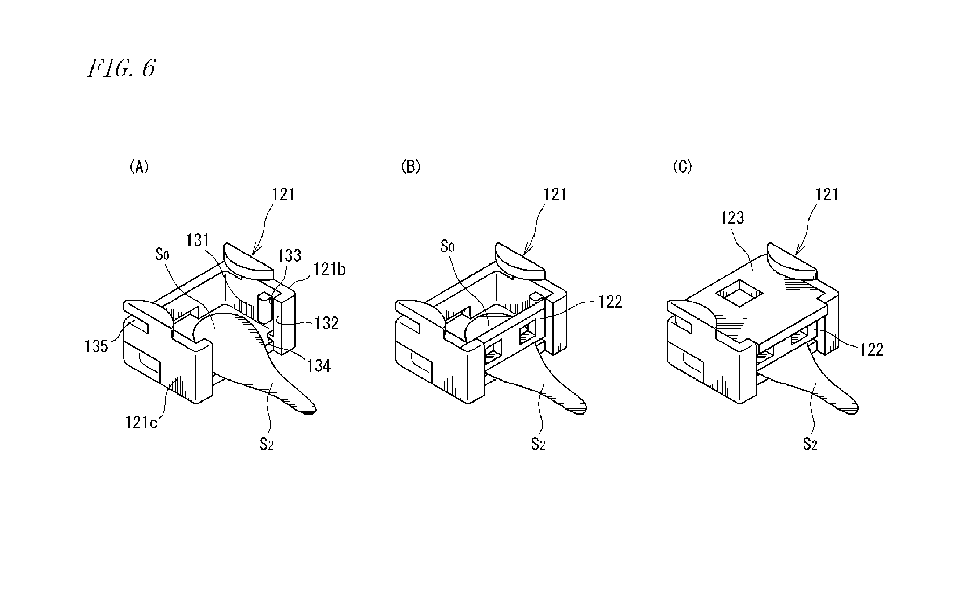

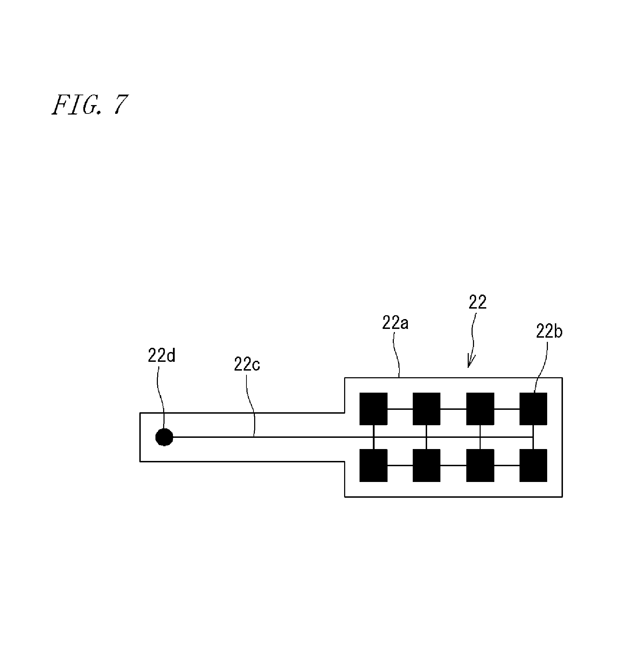

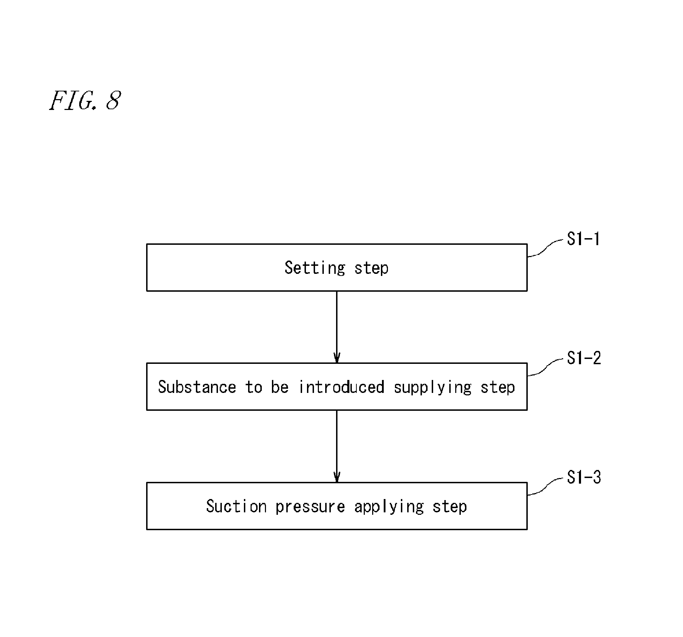

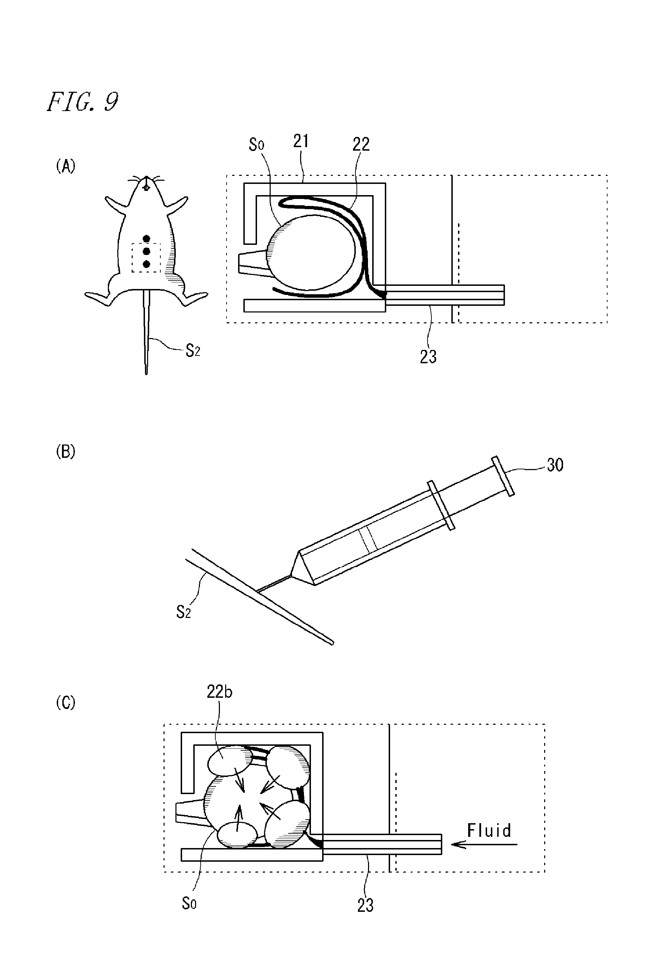

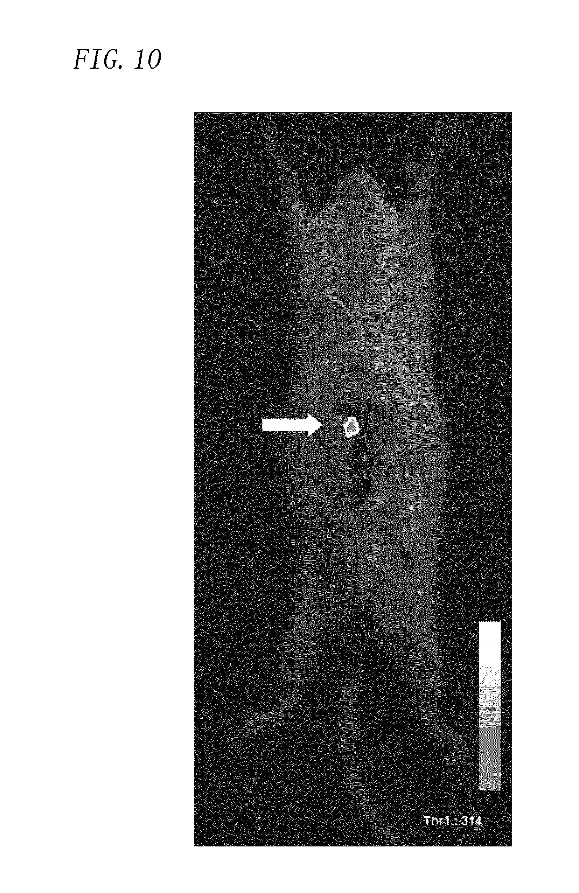

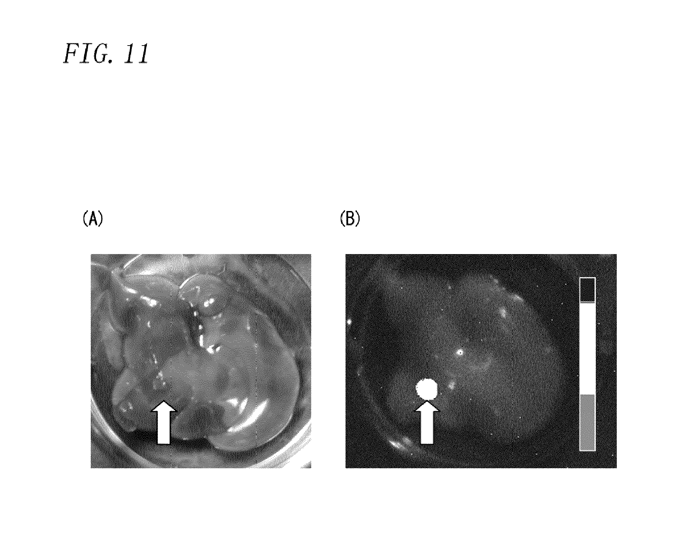

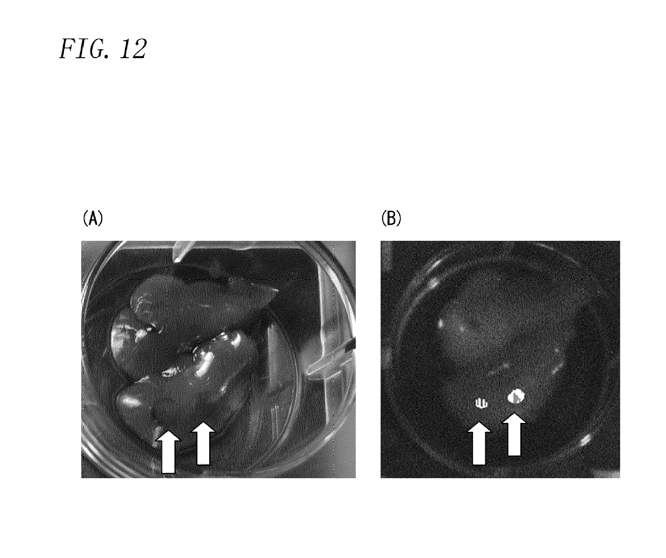

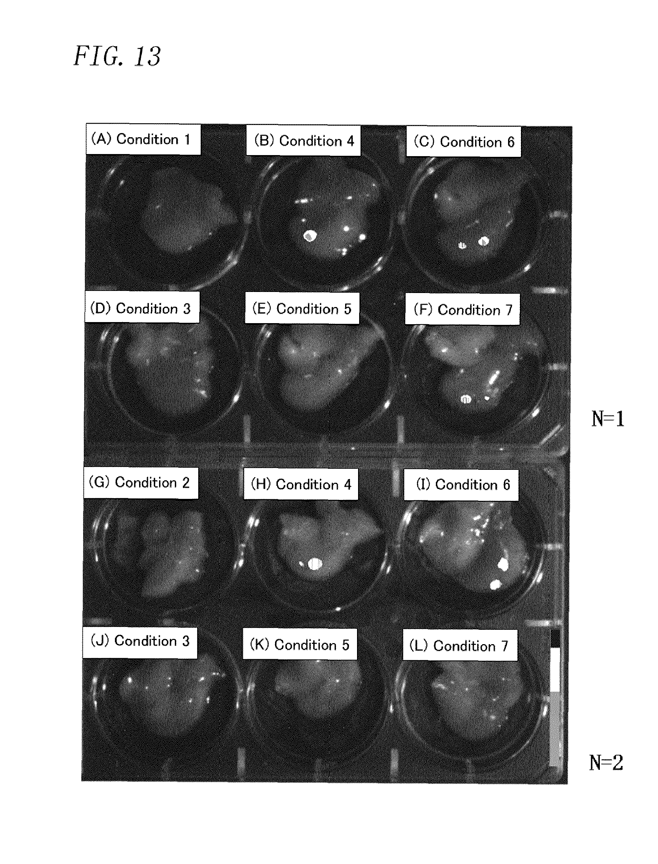

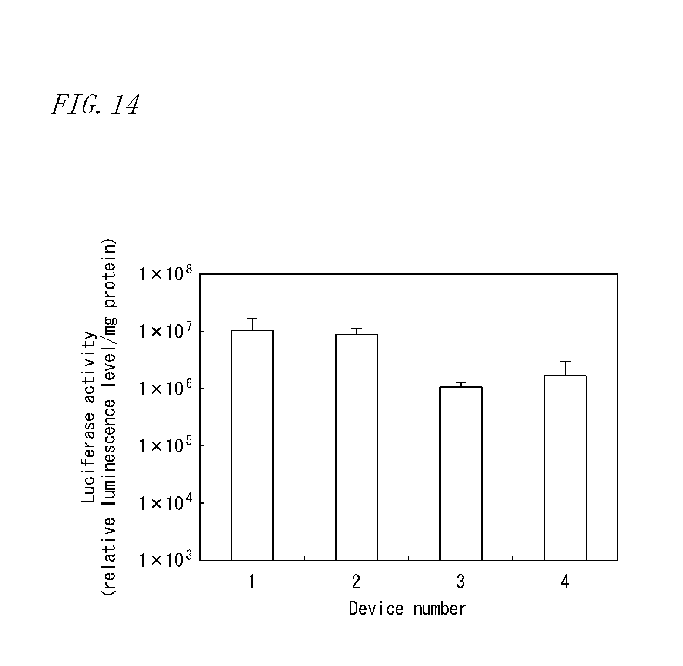

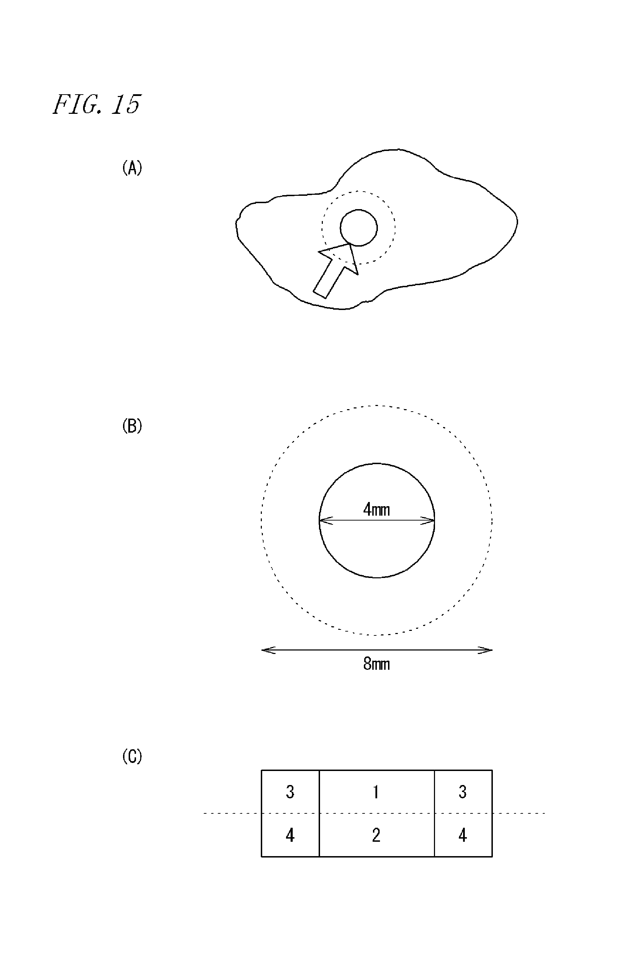

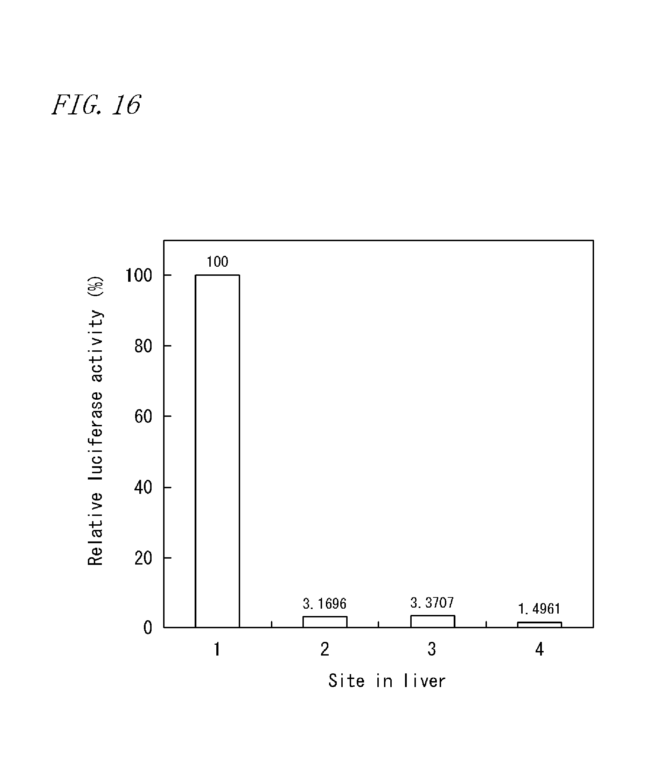

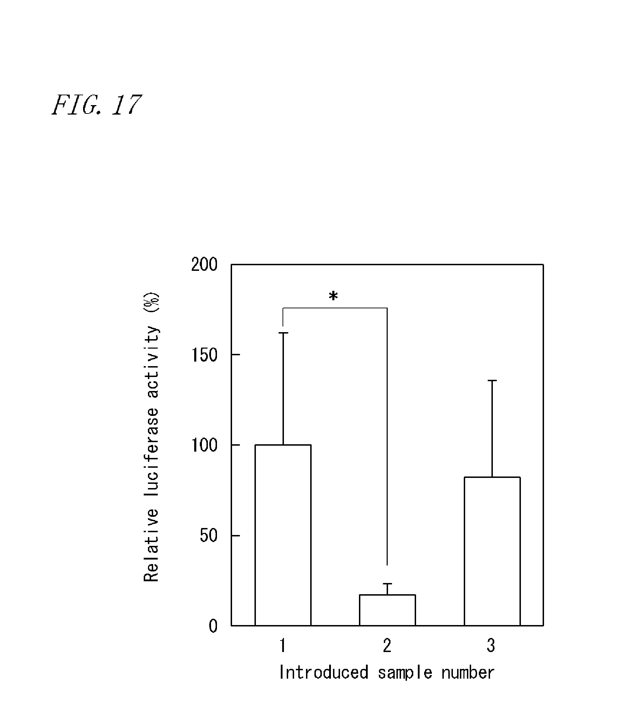

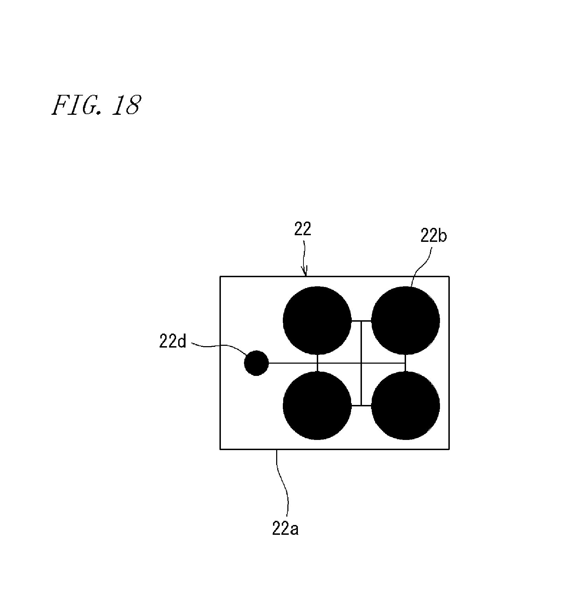

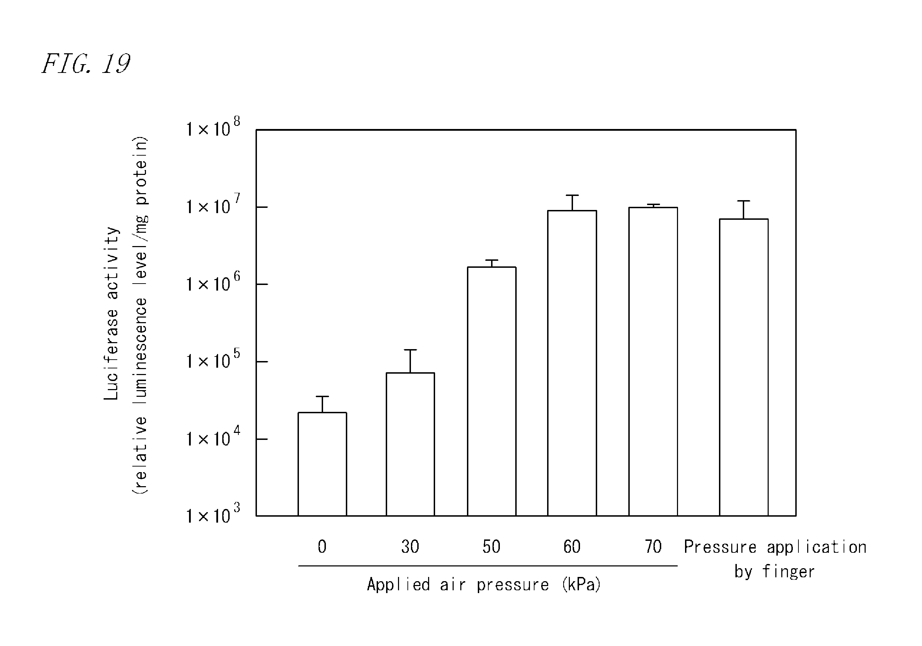

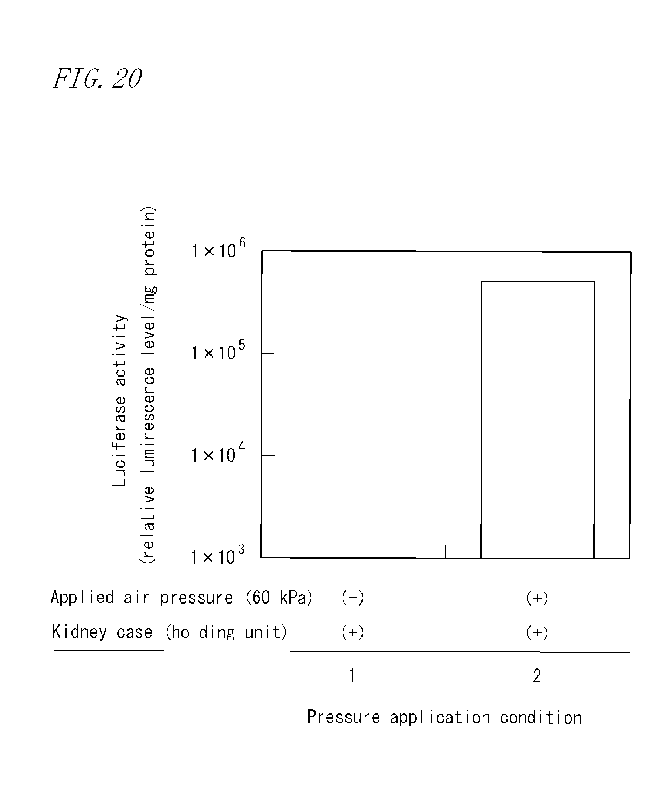

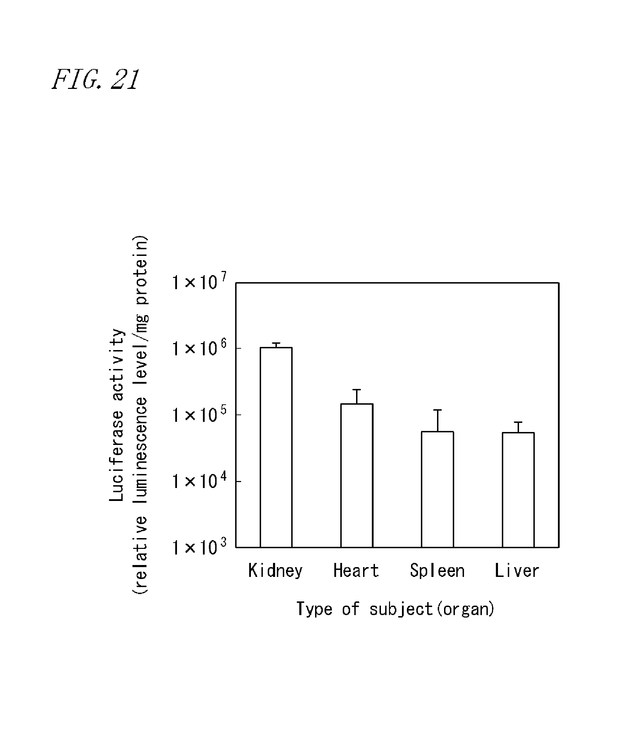

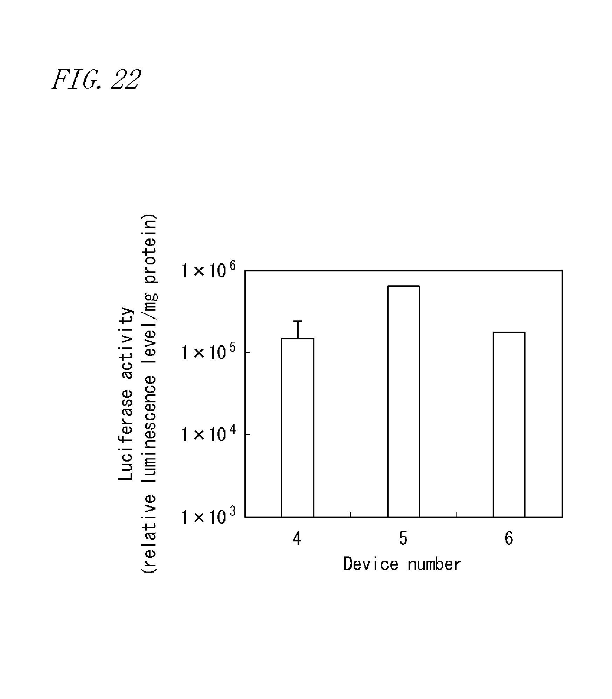

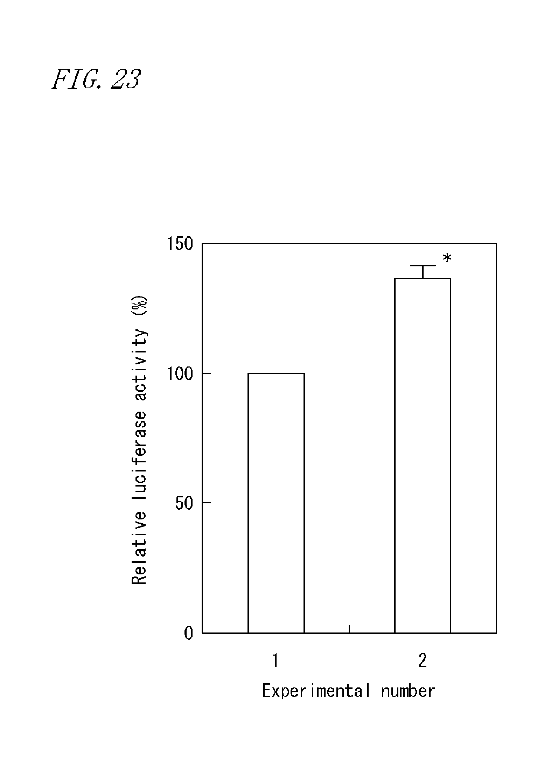

{FIG. 1} FIG. 1 is a block diagram showing an arrangement of a device used in the present invention for delivering a substance to be introduced.
{FIG. 2} FIG. 2 is a schematic explanation view showing an essential part of a device (first delivery device) used in the present invention for delivering a substance to be introduced.
{FIG. 3} FIG. 3 is a schematic explanation view showing an arrangement of a pressure applying unit of the first delivery device.
{FIG. 4} FIG. 4 is a schematic explanation view showing an essential part of a device (second delivery device) used in the present invention for delivering a substance to be introduced.
{FIG. 5} FIG. 5 is an explanatory perspective diagram showing an arrangement of an example of a holding unit of the second delivery device.
{FIG. 6} FIG. 6 is a flowchart showing a procedure for attaching the holding unit of the second delivery device to a kidney.
{FIG. 7} FIG. 7 is a schematic explanation view showing an arrangement of a pressure applying unit of the second delivery device.
{FIG. 8} FIG. 8 is a flowchart showing steps of a method for operating a device for delivering a substance to be introduced and a method for delivering a substance to be introduced according to one embodiment of the present invention.
{FIG. 9} FIG. 9 is a flowchart showing steps of a method for operating a device for delivering a substance to be introduced and a method for delivering a substance to be introduced according to another embodiment of the present invention.
{FIG. 10} FIG. 10 is a drawing-substituting photograph showing a result of an examination in Example 1 of expression sites of luciferase encoded by an injected nucleic acid in a mouse.
{FIG. 11} FIG. 11 is a drawing-substituting photograph showing a result of an examination in Example 2 of expression sites of luciferase encoded by a nucleic acid injected when suction was applied at a single target site in the liver of a mouse.
{FIG. 12} FIG. 12 is a drawing-substituting photograph showing a result of an examination in Example 3 of expression sites of luciferase encoded by an injected nucleic acid when suction was simultaneously applied at two target sites in the liver of a mouse.
{FIG. 13} FIG. 13 is a drawing-substituting photograph showing results of an examination in Test Example 1 of expression sites of luciferase encoded by a nucleic acid injected under various conditions.
{FIG. 14} FIG. 14 is a graph showing a result of an examination in Example 4 on a relationship between the type of device used and luciferase activity.
{FIG. 15} FIG. 15(A) is a schematic explanation view showing the position of a portion of the liver where a suction part was placed in Test Example 2. FIG. 15(B) is an enlarged explanatory diagram of the portion where the suction part shown in FIG. 15(A) was placed. FIG. 15(C) is a schematic explanation view showing sections of a portion which is a target where luciferase activity was measured in Test Example 2.
{FIG. 16} FIG. 16 is a graph showing a result of an examination in Test Example 2 on a relationship between a site in the liver and relative luciferase activity.
{FIG. 17} FIG. 17 is a graph showing a result of an examination in Test Example 3 on a relationship between the type of injection sample and relative luciferase activity.
{FIG. 18} FIG. 18 is a schematic explanation view showing an arrangement of a pressing unit used to apply pressure in Test Example 4.
{FIG. 19} FIG. 19 is a graph showing a result of an examination in Test Example 4 on a relationship between applied air pressure or a manner of applying pressure, and luciferase activity.
{FIG. 20} FIG. 20 is a graph showing a result of an examination in Test Example 5 on a relationship between pressure application conditions and luciferase activity.
{FIG. 21} FIG. 21 is a graph showing results of an examination in Example 5 on a relationship between the type of subject and luciferase activity.
{FIG. 22} FIG. 22 is a graph showing a result of an examination in Example 6 on a relationship between the type of device used and luciferase activity.
{FIG. 23} FIG. 23 is a graph showing a result of an examination in Test Example 6 on a relationship between the type of an introduction ,method and relative luciferase activity.

### MODE FOR CARRYING OUT THE INVENTION

The present inventors have found that, in a subject, by injecting a nucleic acid into a site different from a target site and applying pressure (suction pressure or pressing force) to the target site, the nucleic acid can be locally delivered into the target site, and that a protein encoded by the nucleic acid or a function of the nucleic acid can be expressed at the target site. The present invention has been made based on these findings.

The term "tissue" as used herein refers to a tissue of an animal organ having a vascular path through which a body fluid or a metabolic product is transported, the tissue including an epithelial tissue, a connective tissue, a muscle tissue, a nervous tissue, and the like. Examples of the tissues include a tissue constitutes a kidney, liver, pancreas, stomach, spleen, heart, small intestine, large intestine, lung, uterus, bladder, esophagus, duodenum, brain, eyeball, trachea, skeletal muscle, diaphragm, adrenal gland, prostate gland, ovary, fallopian tube, and the like. The term "vascular path" as used herein refers to a blood vessel, the ureter, a lymphatic vessel, or the like of an animal. The term "vascular path located upstream of a target site" as used herein refers to a vascular path through which a body fluid or a metabolic product is transported to the target site. The term "upstream" refers to a point closer to the starting point of the direction in which a body fluid or a metabolic product flows.

The substance to be introduced is not particularly limited, and includes a nucleic acid, a pharmaceutical, a protein, a peptide or a derivative thereof (hereinafter also referred to as "peptide derivative"), an antibody, a high molecular compound, a metal (for example, a metal nanoparticle, and the like), a semiconductor (for example, a quantum dot, and the like), or a substance composed of a combination thereof, and the like. The term "peptide derivative" encompasses a compound having a peptide backbone. The protein, the peptide, and the peptide derivative are herein collectively referred to as "peptide compound." Among of these substances, the nucleic acid or the peptide compound are preferable, because the nucleic acid or the peptide compound can be efficiently introduced into a cell of a tissue or an organ by the present invention, even though it is generally difficult for the nucleic acid or the peptide compound to pass through a cell membrane.

### 1. Method for operating a device for delivering a substance to be introduced

In one aspect, the present invention is directed to a method (hereinafter referred to as the "method 1") for operating a device for delivering a substance to be introduced, the device including
a supply unit configured to supply the substance to be introduced to a vascular path located upstream of a target site in a tissue existed in a living organism, and
a pressure applying unit configured to apply pressure to the target site in the tissue,
the method including the steps of:
(1-1) causing the supply unit to discharge the substance to be introduced, thereby supplying the substance to be introduced to the vascular path by, and
(1-2) simultaneously with or after the step (1-1), causing the pressure applying unit to generate pressure against the target site in the tissue.

One of the significant features of the method 1 resides in that the substance to be introduced is discharged from the supply unit of the delivery device, thereby being supplied to the vascular path located upstream of the target site in the tissue existed in the living organism. Therefore, according to the method 1, the occurrence of unnecessary damage to the target site in the tissue can be suppressed. One of significant features of the method 1 also resides in that simultaneously with or after the supplying of the substance to be introduced to the vascular path, the pressure applying unit is caused to generate pressure against the target site in the tissue. Therefore, according to the method 1, the substance to be introduced can be locally delivered into the target site to which the pressure is applied. Also, even when a substance having difficulty in passing through a cell membrane is used as the substance to be introduced, the substance can be delivered into the target site.

The living organism includes, for example, an animal having a vascular path through which a body fluid or a metabolic product are transported, and the like. Such animal includes human, non-human mammal, a bird (for example, chicken, quail, and the like), and the like.

Next, an example of the device used in the method 1 for delivering the substance to be introduced will be described with reference to the accompanying drawings. However, the present invention is not intended to be limited to the exemplified delivery device. FIG. 1 is a block diagram showing an arrangement of a device used in the present invention for delivering a substance to be introduced.

The delivery device 1 shown in FIG. 1 has a pressure applying unit 10a which applies pressure to a target site in a tissue existed in a living organism, a supply unit 30 which supplies the substance to be introduced to a vascular path located upstream of the target site in the tissue, and a control unit 40 which controls the pressure applying unit 10a and the supply unit 30.

The delivery device used in the method 1 is broadly classified into a device (first delivery device) which applies suction pressure as the pressure, and a device (second delivery device) which applies pressing force as the pressure. Each of the delivery devices will be described hereinafter.

FIG. 2 is a schematic explanation view showing an essential part of a device (first delivery device) used in the present invention for delivering a substance to be introduced. FIG. 3 is a schematic explanation view showing an arrangement of a pressure applying unit of the first delivery device. The first delivery device 1 has a pressure applying unit 10a which applies suction pressure against a target site S₁ in a tissue S₀ existed in a living organism, a supply unit 30 which supplies the substance to be introduced to a vascular path S₂ located upstream of the target site S₁ of the tissue S₀, and a control unit (not shown) which controls the supply unit 30 and the pressure applying unit 10a.

As shown in FIG. 3, the pressure applying unit 10a has a suction part 11 for making contact with the tissue S₀ and applying suction at the target site S₁, a connection part 12 which connects to a suction device which suctions out air from the suction part 11 or other devices, and a flexible pipe member 13 which connects the suction part 11 and the connection part 12 together. One end of the connection part 12 is connected to the pipe member 13, and the other end is connectable to the suction device which suctions out air from the suction part 11 or other devices (for example, an endoscope, forceps, and the like).

The suction part 11 has a cup-shape and a suction opening 11a for suctioning out air from the suction part 11 (see FIG. 2).

In the pressure applying unit 10a, air in the suction part 11 is suctioned out from the suction opening 11a such that suction is applied at the target site S₁ in the tissue S₀ (see FIG. 2). As a result, the substance to be introduced which has been injected into the vascular path S₂ by the supply unit 30 is delivered into the target site S₁.

The pressure applying unit 10a has a size which allows it to be attached to the suction device which suctions out air from the suction part 11 or other devices (for example, an endoscope, forceps, and the like).

The supply unit 30 has a reservoir part 30a which stores the substance to be introduced, and a discharge part 30b which discharges the substance to be introduced. The discharge part 30b has a needle-like tip which can be inserted into the vascular path S₂ located upstream of the target site S₁ so that the substance to be introduced can be introduced into the vascular path S₂.

The control unit 40 controls the pressure applying unit 10a, and the reservoir part 30a and the discharge part 30b of the supply unit 30. The control unit 40 controls suction of the target site S₁ by the pressure applying unit 10a, transfer of the substance to be introduced from the reservoir part 30a to the discharge part 30b, discharge of the substance to be introduced from the discharge part 30b to the vascular path S₂, and the like.

The delivery device 1 may be configured so that the pressure applying unit 10a and the supply unit 30 are controlled manually in place of using the control unit.

In the delivery device 1, since suction is applied at the target site S₁ by the pressure applying unit 10a, damage to the target site S₁ can be suppressed by using the delivery device 1, compared to the conventional direct injection method. Also, according to the delivery device 1, the substance to be introduced can be delivered into the target site S₁ to which suction pressure is applied, without blocking the flow of blood unlike the conventional method of using a catheter.

Moreover, when the substance to be introduced is delivered into an organ or the like in a living organism, if an insertion opening which allows the pressure applying unit 10a to be inserted is formed in the living organism, the pressure applying unit 10a can be placed at the target site S₁. Therefore, the delivery device 1 allows for less invasive delivery of the substance to be introduced.

FIG. 4 is a schematic explanation view showing an essential part of a device (second delivery device) used in the present invention for delivering a substance to be introduced.

The delivery device 2 of FIG. 4 includes a pressure applying unit 10b which applies pressing force against a target site S₁ in a tissue S₀ existed in a living organism, and a supply unit 30 which supplies the substance to be introduced to a vascular path S₂ located upstream of the target site S₁ of the tissue S₀. The delivery device 2 also has a control unit 40 (personal computer) which controls the pressure applying unit 10b and the supply unit 30.

The pressure applying unit 10b has a holding unit 21 which positionally-fixes and holds the target site S₁ in the tissue S₀ existed in the living organism, a pressing unit 22 which is inflated with a fluid to press the target site S₁, a port 23 which is placed in the living organism to receive the fluid from the outside of the living organism, a flow path unit 24 through which the fluid is supplied from the port 23 to the pressing unit 22, and a fluid supply unit 25 which is connected to the port 23 to supply the fluid to the port 23. The fluid supply unit 25 is composed of a fluid reservoir unit 26 which stores the fluid, a converter 27 which converts an electrical signal from the control unit 40 into a fluid pressure signal, a pressure sensor 28 which detects pressure of the fluid, a needle unit 29 that punctures the living organism to be connected to the port 23, and a holding unit 25a which holds the needle unit 29. The converter 27 is connected to the control unit 40.

The holding unit 21 has a holding unit body 121 which accommodates an organ or the like having the target site S₁, a vascular path side lid portion 122 which suppresses misalignment of the organ or the like in a lateral surface direction of the holding unit body 121 while forming a space for passage of the vascular path S₂ connected to the organ or the like at a lateral surface of the holding unit body 121, and an upper lid portion 123 which suppresses misalignment of the organ or the like in a vertical direction of the holding unit body 121 (see FIG. 5). The holding unit 21 is implantable in the living organism. The holding unit 21 preferably has rigidity in order to efficiently apply pressing force against the target site S₁. Also, the holding unit 21 is preferably does not expand, in order to efficiently apply pressing force against the target site S₁.

The holding unit body 121 includes a bottom portion 121a and lateral portions 121b, 121c, 121d (see FIG. 5). On the inside of each of the lateral portions 121b and 121c of the holding unit body 121, a groove portion 133 into which the vascular path side lid portion 122 is fitted is formed by a lateral surface of a protruding portion 131 extending in the vertical direction and an end portion 132 of those lateral portions 121b and 121c (FIG. 6(A)). On the inside of the end portion 132, a stopper 134 is formed which fixes the position in the vertical direction of the vascular path side lid portion 122 to form a space for passage of the vascular path S₂ connected to the organ or the like (see FIG. 6 (A) and (B)). In an upper portion of each of the lateral portions 121b and 121c of the holding unit body 121, a groove portion 135 is formed in a longitudinal direction of those lateral portions 121b and 121c, so that the upper lid portion 123 can be fitted into the groove portions 135 (see FIG. 6 (A) and (C)).

As shown in FIG. 7, the pressing unit 22 includes a pressing unit body 22a, eight balloons 22b integrated with the pressing unit body 22a, a flow path 22c through which the fluid is supplied to the eight balloons 22b, and a fluid inlet 22d from which the fluid is introduced. The balloon 22b is configured to be inflated by supply of the fluid. As a result, the substance to be introduced which has been injected into the vascular path (blood vessel) S₂ by the supply unit 30 is delivered into the target site S₁ (see FIG. 4).

In the delivery device 2, since the sizes of the holding unit 21 and the port 23 of the pressure applying unit 10b vary depending on, for example, the size of the organ or the like having the target site S₁, the sizes are desirably appropriately determined based on, for example, the size of the organ or the like having the target site S₁.

In the delivery device 2, the supply unit 30 is the same as the supply unit of the delivery device 1.

The control unit 40 controls the pressure applying unit 10b, and the reservoir part 30a and the discharge part 30b of the supply unit 30. The control unit 40 controls application of pressing force by the pressure applying unit 10b, transfer of the substance to be introduced from the reservoir part 30a to the discharge part 30b, discharge of the substance to be introduced from the discharge part 30b, and the like.

When the delivery device 2 is used for, for example, a tissue constitutes a kidney, liver, pancreas, stomach, spleen, heart, small intestine, large intestine, lung, uterus, bladder, esophagus, duodenum, brain, eyeball, trachea, skeletal muscle, diaphragm, adrenal gland, prostate gland, ovary, fallopian tube, or the like, the holding unit 21 may have a shape suitable for holding the tissue.

In the delivery device 2, since the target site S₁ is positionally-fixed by the holding unit 21, the target site S₁ is less likely to be misaligned during application of pressing force against the target site S₁. Therefore, the specificity of delivery of the substance to be introduced can be improved by using the delivery device 2.

Next, the steps of a method (referred to as the "method 1-1") for operating a device for delivering a substance to be introduced in the case where the first delivery device 1 is used, will be described in detail. A flowchart showing steps of the method for operating a device for delivering a substance to be introduced according to one embodiment of the present invention is shown in FIG. 8.

Before the method 1-1 is performed, the pressure applying unit 10a of the delivery device 1 is set at a target site S₁ in a tissue S₀ existed in a living organism, and the supply unit 30 is set at a vascular path S₂ located upstream of the target site S₁ in the tissue S₀ existed in the living organism (see a "setting step S1-1" in FIG. 8)).

In the method 1-1, first, the substance to be introduced is discharged from the supply unit 30, thereby being supplied to the vascular path S₂ (step (1-1) (see a "substance to be introduced supplying step S1-2" in FIG. 8)).

A substance to be introduced-supplying position on the vascular path S₂ can be appropriately set based on the type or stability of the substance to be introduced, the position of the target site S₁, and the like. For example, when the substance to be introduced is a nucleic acid, the substance to be introduced-supplying position is preferably one where a distance between the substance to be introduced-supplying position and the target site S₁ is one which does not cause the nucleic acid to degrade, and to which the substance to be introduced is easily supplied.

In the substance to be introduced supplying step S1-2, since the supplied amount of the substance to be introduced varies depending on the type of the substance to be introduced, the type of the target site S₁, and the like, the supplied amount of the substance to be introduced cannot be non-specifically determined. Therefore, the supplied amount of the substance to be introduced is preferably determined based on the type of the substance to be introduced, the type of the target site S₁, and the like.

Simultaneously with or after the substance to be introduced supplying step S1-2, suction is applied at the target site S₁ of the tissue S₀ by the pressure applying unit 10a so that a suction force is generated with respect to the target site S₁ (step (1-2) (see a "suction pressure applying step S1-3" in FIG. 8)).

In the suction pressure applying step S1-3, since the magnitude of the suction pressure varies depending on the amount of the substance to be introduced which is delivered into the target site S₁, an inner diameter D₁, an outer diameter D₂, and an inner wall height h of the suction part 11, and an inner diameter d of the suction opening 11a, the magnitude of the suction pressure cannot be non-specifically determined. Therefore, the magnitude of the suction pressure is preferably determined based on the amount of the substance to be introduced which is delivered into the target site S₁, the inner diameter D₁, the outer diameter D₂, and the inner wall height h of the suction part 11, and the inner diameter d of the suction opening 11a. The magnitude of the suction pressure is typically 10 kPa to 80 kPa.

In the method 1-1, the suction pressure applying step S1-3 may be performed simultaneously with or after the substance to be introduced supplying step S1-2. The timing at which the suction pressure applying step S1-3 is performed may be appropriately set based on the distance between the substance to be introduced-supplying position and the target site S₁, and the like.

In the method 1-1, since suction is applied at the target site S₁ by the pressure applying unit 10a, the substance to be introduced can be specifically delivered into the target site S₁.

Next, the steps of a method (referred to as the "method 1-2") for operating a device for delivering a substance to be introduced, when the second the delivery device 2 is used, will be described in detail. A flowchart showing steps of a method for delivering a substance to be introduced according to another embodiment of the present invention is shown in FIG. 9.

Before the method 1-2 is performed, the pressure applying unit 10b of the delivery device 2 is set at a target site S₁ of a tissue S₀ existed in a living organism, and the supply unit 30 is set at a vascular path S₂ located upstream of the target site S₁ of the tissue S₀ in the living organism (see (A) in FIG. 9). At this time, the organ having the target site S₁ is accommodated in the holding unit 21, and the pressing unit 22 is arranged to surround the organ.

In the method 1-2, first, the substance to be introduced is discharged from the supply unit 30 to be supplied to the vascular path S₂ (step (1-1) (also referred to as the "substance to be introduced supplying step"), see (B) in FIG. 9).

As in the method 1-1, the supplied amount of the substance to be introduced is preferably determined based on the type of the substance to be introduced, the type of the target site S₁, and the like. Also, as in the method 1-1, a substance to be introduced-supplying position on the vascular path S₂ is supplied can be appropriately set based on the type or stability of the substance to be introduced, the position of the target site S₁, and the like.

Simultaneously with or after the substance to be introduced supplying step, a fluid is supplied to the balloon 22b of the pressure applying unit to inflate the balloon 22b, whereby pressing force against the target site S₁ of the tissue S₀ of the organ which is in contact with the balloon 22b is generated (step (1-2) (also referred to as the "pressing force applying step"), see (C) in FIG. 9).

The amount of the fluid supplied to the balloon 22b is proportional to pressing force against the target site S₁. Furthermore, the pressing force against the target site S₁ is proportional to the amount of the substance to be introduced to the target site S₁. Therefore, in the pressing force applying step, the amount of the fluid supplied to the balloon 22b is preferably determined based on the amount of the substance to be introduced into the target site S₁. Typically, the amount of the fluid is preferably adjusted so that the magnitude of the pressing force is 30 kPa to 80 kPa.

In the method 1-2, the pressing force applying step may be performed simultaneously with or after the substance to be introduced supplying step. The timing at which pressing force applying step is performed can be appropriately set based on the distance between the substance to be introduced-supplying position and the target site S₁, and the like.

### 2. Method for delivering a substance to be introduced (1) Method for delivering a substance to be introduced into a target site in a tissue existed in a non-human mammal

According to another aspect, the present invention is directed to a method (hereinafter referred to as the "method 2") for delivering a substance to be introduced into a target site in a tissue existed in a non-human mammal,
the method including the steps of:
(2-1) supplying the substance to be introduced into a vascular path located upstream of the tissue; and
(2-2) simultaneously with or after the step (2-1), applying pressure to the target site in the tissue, thereby delivering the substance to be introduced into the target site.

One of the significant features of the method 2 resides in that the substance to be introduced is supplied to the vascular path located upstream of the target site in the tissue existed in the non-human mammal, and that simultaneously with or after the supply of the substance to be introduced into the vascular path, the pressure is applied to the target site in the tissue. Therefore, the method 2 exhibits the same advantageous effects as those of the method 1.

The non-human mammal includes, for example, monkey, pig, dog, horse, mouse, rabbit, rat, cow, sheep, guinea pig, hamster, and the like.

The method 2 is broadly classified into a method (referred to as the "method 2-1") of using suction pressure as the pressure applied to the target site and a method (referred to as the "method 2-2") of using pressing force as the pressure applied to the target site.

In a same manner as the method 1-1, the method 2-1 can be performed, for example, by performing the steps of FIG. 8 with the use of the delivery device 1 of FIG. 1. Therefore, when the delivery device 1 of FIG. 1 is used, before the method 2-1 is performed, at least the suction part 11 of the pressure applying unit 10a of the delivery device 1 is set at the target site S₁ of the tissue S₀ in the living organism, and the supply unit 30 is set at the vascular path S₂ located upstream of the target site S₁ of the tissue S₀ in the living organism (see the "setting step S1-1" in FIG. 8).

In the method 2-1, first, the substance to be introduced is supplied to the vascular path S₂ located upstream of the tissue S₀ of the non-human mammal (step (2-1) (see the "substance to be introduced supplying step S1-2" in FIG. 8)).

The supplied amount of the substance to be introduced is preferably determined based on the type of the substance to be introduced, the type of the target site S₁, and the like.

A substance to be introduced-supplying position on the vascular path can be appropriately set based on the type or stability of the substance to be introduced, the position of the target site S₁, and the like. The substance to be introduced-supplying position is preferably one where a distance between the substance to be introduced-supplying position and the target site S₁ is one which allows the substance to be introduced to be stably maintained, and to which the substance to be introduced is easily supplied.

The supplied amount of the substance to be introduced can be determined based on the type of the substance to be introduced, the type of the target site S₁, and the like.

Next, simultaneously with or after the step (2-1), pressure (suction pressure) is applied to the target site S₁ of the tissue S₀ so that the substance to be introduced is delivered into the target site S₁ (step (2-2), see the "suction pressure applying step S1-3" in FIG. 8).

The magnitude of the suction pressure is preferably determined based on the amount of the substance to be introduced which is delivered into the target site S₁, and the like. The magnitude of the suction pressure is typically 10 kPa to 80 kPa.

In the method 2-1, the suction of the target site S₁ in the step (2-2) may be performed simultaneously with or after the step (2-1). The timing at which step (2-2) is performed can be appropriately set based on the distance between the substance to be introduced-supplying position and the target site S₁, and the like.

On the other hand, in a same manner as the method 1-2, the method 2-2 can be performed, for example, by performing the steps of FIG. 9 with the use of the delivery device 2 of FIG. 4. Therefore, when the delivery device 2 of FIG. 4 is used, before the method 2-2 is performed, the pressure applying unit 10b of the delivery device 2 is set at the target site S₁ of the tissue S₀ in the living organism, and the supply unit 30 is set at the vascular path S₂ located upstream of the target site S₁ of the tissue S₀ in the living organism (see (A) in FIG. 9). At this time, the organ having the target site S₁ is accommodated in the holding unit 21, and the pressing unit 22 is arranged to surround the organ.

In the method 2-2, first, the substance to be introduced is supplied to the vascular path S₂ located upstream of the tissue S₀ (see (B) in FIG. 9).

The supplied amount of the substance to be introduced is preferably determined based on the type of the substance to be introduced, the type of the target site S₁, and the like.

A substance to be introduced-supplying position on the vascular path S₂ can be appropriately set based on the type or stability of the substance to be introduced, the position of the target site S₁, and the like. The substance to be introduced-supplying position is preferably one where a distance between the substance to be introduced-supplying position and the target site S₁ is one which allows the substance to be introduced to be stably maintained, and to which the substance to be introduced is easily supplied.

In the method 2-2, next, in the step (2-2), the target site S₁ is pressed while the tissue S₀ is positionally-fixed (see (C) in FIG. 9). As a result, pressing force can be applied to the target site S₁, whereby the substance to be introduced can be delivered into the target site S₁.

In the method 2-2, when the tissue S₀ is a tissue of an organ, in step (2-2) the target site S₁ is preferably pressed while the tissue S₀ is positionally-fixed by accommodating the organ in a case capable of accommodating the organ.

The magnitude of pressing force against the target site S₁ is preferably determined based on the amount of the substance to be introduced into the target site S₁. The magnitude of pressing force is typically 30 kPa to 80 kPa.

In the method 2-2, the pressing of the target site S₁ in the step (2-2) may be performed simultaneously with or after the step (2-1). The timing at which step (2-2) is performed can be appropriately determined based on the distance between the substance to be introduced-supplying position and the target site S₁, and the like.

This method for delivering the substance to be introduced can be applied to human in addition to non-human animals. As a result, various substances to be introduced such as a nucleic acid, a pharmaceutical, a peptide compound, an antibody, a high molecular compound, a metal (for example, a metal nanoparticle, and the like), a semiconductor (for example, a quantum dot, and the like), a substance composed of a combination thereof, and the like, can be locally delivered into a target site in therapy or prevention of human diseases, and these substances to be introduced can be locally delivered into a target site in diagnosis in human, and the like.

### (2) Method for delivering a substance to be introduced into a target site in a subject derived from a living organism

According to still another aspect, the present invention is directed to a method (hereinafter referred to as the "method 3") for delivering a substance to be introduced into a target site in a subject derived from a living organism,
the method including the steps of:
(3-1) supplying the substance to be introduced to a site different from the target site in the subject; and
(3-2) simultaneously with or after the step (3-1), applying pressure to the target site in the subject, thereby delivering the substance to be introduced into the target site.

One of the significant features of the method 3 resides in that the substance to be introduced is supplied to the site different from the target site in the subject derived from the living organism, and that simultaneously with or after the supply of the substance to be introduced into the site different from the target site in the subject derived from the living organism, pressure is applied to the target site in the tissue. Therefore, the method 3 exhibits the same advantageous effects as those of the method 1.

The living organism includes, for example, human, a non-human mammal, a bird (for example, chicken, quail, and the like), and the like.

Examples of the subject derived from the living organism include a tissue isolated from the living organism (for example, a tissue that constitutes a kidney, liver, pancreas, stomach, spleen, heart, small intestine, large intestine, lung, uterus, bladder, esophagus, duodenum, brain, eyeball, trachea, skeletal muscle, diaphragm, adrenal gland, prostate gland, ovary, fallopian tube, or the like), an organ isolated from the living organism (for example, a kidney, liver, pancreas, stomach, spleen, heart, small intestine, large intestine, lung, uterus, bladder, esophagus, duodenum, brain, eyeball, trachea, skeletal muscle, diaphragm, adrenal gland, prostate gland, ovary, fallopian tube, and the like), an artificial tissue composed of a cell of the living organism (for example, an aggregate of a single type of cells derived from somatic cells or stem cells, an aggregate of a cell population of a single type of cells derived from somatic cells or stem cells and a scaffold for the cells, and the like), an artificial organ composed of a cell of the living organism (for example, an aggregate of multiple types of cells derived from somatic cells or stem cells, an aggregate of a cell population of multiple types of cells derived from somatic cells or stem cells and a scaffold for the cells, and the like), and the like. These subjects desirably have a vascular path connected to the target site, but the present invention is not limited to this.

The method 3 is broadly classified into a method (referred to as the "method 3-1") of using suction pressure as the pressure applied to the target site and a method (referred to as the "method 3-2") of using pressing force as the pressure applied to the target site.

In a same manner as the methods 1-1 and 2-1, the method 3-1 can be performed, for example, by performing the steps of FIG. 8 with the use of the delivery device 1 of FIG. 1. Therefore, when the delivery device 1 of FIG. 1 is used, before the method 3-1 is performed, at least the suction part 11 of the pressure applying unit 10a of the delivery device 1 is set at the target site S₁, and the supply unit 30 is set at a site different from the target site S₁ (see the "setting step S1-1" in FIG. 8).

In the method 3-1, first, the substance to be introduced is supplied to a site in the subject different from the target site S₁ (step (3-1) (see the "substance to be introduced supplying step S1-2" in FIG. 8)).

The supplied amount of the substance to be introduced is preferably determined based on the type of the substance to be introduced, the type of the target site S₁, and the like.

A substance to be introduced-supplying position in the subject can be appropriately set based on the type or stability of the substance to be introduced, the position of the target site S₁, and the like. The substance to be introduced-supplying position is preferably one where a distance between the substance to be introduced-supplying position and the target site S₁ is one which allows the substance to be introduced to be stably maintained, and to which the substance to be introduced is easily supplied.

The supplied amount of the substance to be introduced can be determined based on the type of the substance to be introduced, the type of the target site S₁, and the like.

Next, simultaneously with or after the step (3-1), pressure (suction pressure) is applied to the target site S₁ of the subject so that the substance to be introduced is delivered into the target site S₁ (step (3-2), see the "suction pressure applying step S1-3" in FIG. 8).

The magnitude of the suction pressure is preferably determined based on the amount of the substance to be introduced which is delivered into the target site S₁, and the like. The magnitude of the suction pressure is typically 10 kPa to 80 kPa.

In the method 3-1, the suction of the target site S₁ in the step (3-2) may be performed simultaneously with or after the step (3-1). The timing at which step (3-2) is performed can be appropriately set based on the distance between the substance to be introduced-supplying position and the target site S₁, and the like.

On the other hand, the method 3-2 is preferable when, as the subject derived from the living organism, a tissue of a muscle or the like isolated from the living organism, an organ isolated from the living organism, or an artificial organ composed of cells of the living organism, is used. In this case, in a same manner as the method 1-2, the method 3-2 can be performed, for example, by performing the steps of FIG. 9 with the use of the delivery device 2 of FIG. 4. In this case, before the method 3-2 is performed, the pressure applying unit 10b of the delivery device 2 is set at the target site S₁ of the subject derived from the living organism, and the supply unit 30 is set at a site different from the target site S₁ of the subject (see (A) in FIG. 9).

Thereafter, in the subject derived from the living organism, the substance to be introduced is supplied to the site different from the target site S₁ (see (B) in FIG. 9).

The supplied amount of the substance to be introduced is preferably determined based on the type of the substance to be introduced, the type of the target site S₁, and the like.

A substance to be introduced-supplying position in the subject is preferably a site where substances are transported to/from the target site S₁, in order to improve the efficiency of delivery of the substance to be introduced. Examples of such a site where the substance is transported to/from the target site S₁ include vascular paths, artificially produced vascular paths, and the like. The substance to be introduced-supplying position is preferably set based on the type or stability of the substance to be introduced, the position of the target site S₁, and the like. Specifically, the substance to be introduced-supplying position is preferably one where a distance between the substance to be introduced-supplying position and the target site S₁ is one which allows the substance to be introduced to be stably maintained, and to which the substance to be introduced is easily supplied.

In the method 3-2, next, in the step (3-2), the target site S₁ is pressed while being positionally-fixed (see (C) in FIG. 9). As a result, pressing force can be applied to the target site S₁, whereby the substance to be introduced can be delivered into the target site S₁.

In the method 3-2, when the subject is a tissue such as a muscle or the like isolated from the living organism, an organ isolated from the living organism, or an artificial organ composed of cells of the living organism, the target site S₁ is positionally-fixed by accommodating the organ in a case capable of accommodating the organ and thereby binding the organ, in the step (3-2).

The magnitude of pressing force against the target site S₁ is preferably determined based on the amount of the substance to be introduced into the target site S₁. The magnitude of pressing force is typically 30 kPa to 80 kPa.

In the method 3-2, the pressing of the target site S₁ in the step (3-2) may be performed simultaneously with or after the step (3-1). The timing at which step (3-2) is performed can be appropriately set based on the distance between the substance to be introduced-supplying position and the target site S₁, and the like.

As described above, by the method for operating a device for delivering a substance to be introduced of the present invention and the method for delivering a substance to be introduced of the present invention, the substance to be introduced can be efficiently locally delivered into a target site. Therefore, the method for operating a device for delivering a substance to be introduced of the present invention and the method for delivering a substance to be introduced of the present invention are useful for locally delivering various substances to be introduced such as a nucleic acid, a pharmaceutical, a peptide compound, an antibody, a high molecular compound, a metal (for example, a metal nanoparticle, and the like), a semiconductor (for example, a quantum dot, and the like), a substance composed of a combination thereof, and the like, into a target site in therapy or prevention of diseases, locally delivering these substances to be introduced into a target site in diagnosis, and the like.

### Examples

The present invention will be more specifically described hereinafter by way of Examples, but the present invention is not intended to be restricted by only the Examples.

### {Example 1}

The abdominal cavity of an anesthetized mouse was opened by midline incision to expose the liver. Thereafter, the suction part 11 of the pressure applying unit 10a of the delivery device 1 for delivering a substance to be introduced (see FIG. 2 and FIG. 3) was placed at a target site in the liver. Here, the inner diameter D₁ of the suction part 11 of the pressure applying unit 10a is 3 mm, the outer diameter thereof D₂ being 5 mm, and the inner wall height thereof h being 3 mm, respectively. In addition, the inner diameter d of the suction opening 11a of the suction part 11 is 1 mm.

Next, by using the supply unit 30 of the delivery device 1, 100 µg of plasmid pCMV-LUC was injected into a blood vessel in the tail of a mouse. Immediately after injection, by using the pressure applying unit 10a of the delivery device 1, suction was applied at the target site in the liver in an amount equivalent to 2.5 mL for 5 seconds. Thereafter, the abdominal cavity of the mouse was closed, and the mouse was reared for six hours. In addition, the plasmid pCMV-LUC is a plasmid which is produced from a plasmid having a nucleic acid encoding luciferase (GenBank accession number: U47296, pGL3-control, manufactured by Promega Corporation) (see T. Nomura et al., "Gene expression and antitumor effects following direct interferon (IFN)-y gene transfer with naked plasmid DNA and DC-chol liposome complexes in mice," Gene Therapy, January 1999, Vol. 6, No. 1, p. 121-129).

Next, the mouse was anesthetized. Thereafter, 200 µL of a luciferin solution containing luciferin, which is the substrate of luciferase, was administered into the abdominal cavity. The luciferin solution was prepared by dissolving luciferin in saline to a concentration of 25 mg/mL according to instructions for a commercially available reagent (trade name: PicaGene PGL1500, manufactured by TOYO B-net, CO., LTD.). The mouse after the administration was placed in an imaging apparatus (trade name: NightOWL NC320, manufactured by BERTHOLD TECHNOLOGIES GmbH & Co. KG). An image of luminescence based on luciferase was captured by a camera and examined with naked eyes. The result of the examination in Example 1 of expression sites of luciferase encoded by the nucleic acid injected in the mouse is shown in FIG. 10. In the figure a site indicated by an arrow indicates a site where luminescence based on the luciferase was observed.

From the results shown in FIG. 10, luminescence based on the luciferase in the liver of the mouse can be observed by the naked eye. Therefore, from the result, it can be seen that, in a subject, a nucleic acid injected into a site different from a target site was locally delivered into the target site to which pressure (suction pressure) was applied, and in addition, at the target site, a protein encoded by the nucleic acid was expressed.

### {Examples 2 and 3}

An image of luminescence based on the luciferase was captured by a camera and examined with naked eyes in the same manner as in Example 1, except that after a solution containing luciferin was administered to a mouse, the abdominal cavity of the mouse was opened to eviscerate the liver (Example 2). The result of an examination in Example 2 of expression sites of luciferase encoded by the injected nucleic acid when suction was applied at a single target site in the liver of the mouse was shown in FIG. 11. In the figure, (A) shows a result of directly viewing the liver of the mouse, and (B) shows a result of the examination of the expression site of the luciferase.

In addition, an image of luminescence based on the luciferase was captured by a camera and examined with naked eyes in the same manner as in Example 1, except that suction was simultaneously applied at two target sites in the liver of the mouse (Example 3). The result of an examination in Example 3 of expression sites of luciferase encoded by the injected nucleic acid when suction was applied at the two target sites in the liver of the mouse is shown in FIG. 12. In the figure, (A) shows a result of directly viewing the liver of the mouse, and (B) shows a result of the examination of expression sites of the luciferase.

From the results shown in FIG. 11 (A) and (B), when suction was applied at the single target site, luminescence based on the luciferase was observed with naked eyes only at the target site. From the results shown in FIG. 12 (A) and (B), when suction was applied simultaneously at the two target sites, luminescence based on the luciferase was observed with naked eyes at both of the two target sites. Therefore, from these results, it can be seen that, when a plurality of target sites exist, a nucleic acid injected to a site different from the target sites can be delivered into each of the target sites in a subject by applying suction at each of the target sites.

### { {Test Example 1}

An image of luminescence based on the luciferase was captured by a camera and examined with naked eyes in the same manner as in Example 1, except that the nucleic acid was injected under various conditions shown in Table 1. In the table, the diameter D₁ means the inner diameter D₁ of the suction part 11 of the pressure applying unit 10a in the delivery device 1 of FIG. 2. The results of the examination in Test Example 1 of expression sites of luciferase encoded by the nucleic acid injected under the various conditions are shown in FIG. 13.

**{Table 1}**

| Condition number | With or without laparotomy | Presence or absence of pcmv-Luc | Suction scheme | Diameter D₁(mm) | Suction amount (ml-equivalent value) |
|---|---|---|---|---|---|
| 1 | - | - | - | - | - |
| 2 | - | + | - | - | - |
| 3 | + | + | - | - | - |
| 4 | + | + | Only one site | 4 | 2.5 |
| 5 | + | + | Only one site | 2 | 2.5 |
| 7 | + | + | Two sites simultaneously | 3 | 2.5 |
| 7 | + | + | Two sites sequentially | 3 | 2.5 |

From the results shows in FIG. 13, when the delivery device having the suction part 11 was used (conditions 4, 6, and 7 in FIG. 13), luminescence based on the luciferase can be observed at the target site with naked eyes. When the delivery device having the suction part 11 having a diameter D₁ of 2 mm was used, luminescence based on the luciferase was not clearly observed with naked eyes, but in view of a result of Example 4 described below, it is considered that the nucleic acid was actually delivered into the target site.

On the other hand, from the results shown in FIG. 13, when suction was not performed (conditions 2 and 3 in FIG. 13), luminescence of the luciferase was not observed.

Therefore, these results suggest that a nucleic acid can be more efficiently delivered into the target site, by using the delivery device having the suction part 11 to apply suction at a target site.

### {Example 4}

The abdominal cavity of an anesthetized mouse was opened by midline incision to expose a kidney. Thereafter, the suction part 11 of the pressure applying unit 10a of the delivery device 1 (see FIG. 2 and FIG. 3) was placed at a target site in a kidney. As the delivery device 1, delivery devices numbered 1 to 4 in Table 2 were each used. The inner wall height h of the suction part 11 was 3 mm.

**{Table 2}**

| Device number | Inner diameter D₁(mm) | Outer diameter D₂(mm) | Suction amount (mL) | Suction scheme |
|---|---|---|---|---|
| 5 | 2 | 3 | 3 | Only one site |
| 6 | 2 | 3 | 3 | Only one site |
| 7 | 3 | 4 | 3 | Only one site |
| 7 | 3 | 4 | 3 | Three sites sequentially |

Next, by using the supply unit 30 of the delivery device 1, 100 µg of the pCMV-Luc was injected into a blood vessel in the tail of the mouse. Immediately after injection, by using the pressure applying unit 10a of the delivery device 1, suction was applied at the target site in a kidney for 5 seconds under conditions shown in Table 2. Thereafter, the abdominal cavity of the mouse was closed, and the mouse was reared for six hours.

Next, the mouse was euthanized under anesthesia, the kidney was eviscerated, and the mass of the kidney was measured. Thereafter, the kidney was placed in a lysis solution (composition: 0.05 mass% Triton (registered trademark) X-100, 2 mM ethylenediaminetetraacetic acid, and 0.1 M Tris-HCl) in a ratio of 5 mL of the lysis solution per gram of the kidney. Thereafter, by using a homogenizer, the resulting mixture was homogenized and dissolved at 4°C to give a pulverized tissue solution. Next, the pulverized tissue solution was centrifuged (10000 x g, 10 min, and 4°C) to collect a supernatant. One-hundred microliters of the luciferin-containing solution was added to 10 µL of the collected supernatant, followed by mixing to give a mixture. Thereafter, a luminometer (trade name: Lumat LB 9507, manufactured by EG&G BERTHOLD) was used to measure the luciferase activity of the mixture. A result of an examination in Example 4 on a relationship between the type of the device used and the luciferase activity is shown in FIG. 14.

From the result shown in FIG. 14, when the suction part having the same size was used, the luciferase activity obtained when the amount of suction was 5 mL was higher than the luciferase activity obtained when the amount of suction was 2.5 mL. Therefore, this result suggests that the amount of a nucleic acid to be delivered can be set by adjusting the amount of suction.

It can also be seen that when the amount of suction was the same, the luciferase activity obtained when the suction part having an inner diameter D₁ of 4 mm and an outer diameter D₂ of 6 mm was used was higher than the luciferase activity obtained when the suction part having an inner diameter D₁ of 2 mm and an outer diameter D₂ of 3 mm was used. Therefore, this result suggests that the amount of a nucleic acid to be delivered can be set by adjusting the size of the suction part.

It can also been seen that when the suction part having the same size was used and the amount of suction was the same, the level of the luciferase activity obtained when the number of sites to which suction pressure was applied was three was higher than the luciferase activity obtained when the number of sites to which suction pressure was applied was one. Therefore, this result suggests that the amount of a nucleic acid to be delivered or the site to which a nucleic acid is to be delivered can be set by repeatedly performing suction.

### {Test Example 2}

The abdominal cavity of an anesthetized mouse was opened by midline incision to expose the liver. Thereafter, the suction part 11 of the pressure applying unit 10a of the delivery device 1 (see FIG. 2 and FIG. 3) was placed at a target site in the liver. The inner diameter D₁ of the suction part 11 of the pressure applying unit 10a was 4 mm, the outer diameter thereof D₂ being 8 mm, and the inner wall height h being 3 mm. A position of a portion of the liver where the suction part was placed in Test Example 2 is shown in FIG. 15(A). In the figure, an arrow corresponds to a portion where the suction opening was placed. A dashed line portion corresponds to a portion with which a wall portion of the suction part was in contact. An enlarged explanatory diagram of the portion where the suction part shown in FIG. 15(A) was placed is shown in FIG. 15(B).

Next, by using the supply unit 30 of the delivery device 1, 100 µg of the pCMV-Luc was injected into a blood vessel in the tail of the mouse. Immediately after injection, by using the pressure applying unit 10a of the delivery device 1, suction was applied at the target site in the liver in an amount equivalent to 2.5 mL for 5 seconds. Thereafter, the abdominal cavity of the mouse was closed, and the mouse was reared for six hours.

Next, the mouse was euthanized under anesthesia. The portion of the liver where the suction part 11was placed was hollowed out in the shape of a cylinder having a diameter of 8 mm and a height of about 3 to 4 mm. The obtained section was divided into four sections. Thereafter, the mass of each of the sections was measured. Each of the sections was used as a subject of which the luciferase activity was measured. The sections of the portion which are the subjects of which the luciferase activity was measured in Test Example 2 are shown in FIG. 15(C).

Each of the sections was added to the lysis solution in a ratio of 25 mL of the lysis solution per gram of the section. By using a homogenizer, the resulting mixture was homogenized and dissolved at 4°C to give a pulverized tissue solution. Next, the pulverized tissue solution was centrifuged (10000 x g, for 10 min at 4°C), and the supernatant was collected. One-hundred micrliters of the luciferin-containing solution was added to 10 µL of the collected supernatant, followed by mixing to give a mixture. Thereafter, the luciferase activity of the mixture was measured by using the luminometere. A result of an examination in Test Example 2 on a relationship between the site in the liver and the relative luciferase activity is shown in FIG. 16.

From the results shown in FIG. 16, of the portion which was in contact with the suction opening, the level of the luciferase activity was highest in the portion (section 1) located at a depth of about 1.5 mm or less. This result suggests that a nucleic acid was locally delivered into a surface of a suction site.

### {Test Example 3}

The abdominal cavity of an anesthetized mouse was opened by midline incision to expose the liver. Thereafter, the suction part 11 of the pressure applying unit 10a of the delivery device 1 (see FIG. 2 and FIG. 3) was placed at a target site in the liver. The inner diameter D₁ of the suction part 11 of the pressure applying unit 10a was 4 mm, the outer diameter D₂, being 6 mm and the inner wall height h being 3 mm.

Next, using the supply unit 30 of the delivery device 1, 18 µg of pCMV-Luc alone (injection sample No. 1), a combination of 18 µg of pCMV-Luc and 10 µg of siRNA (siRNA-LUC) for a nucleic acid encoding luciferase (injection sample No. 2), or a combination of 18 µg of pCMV-Luc and 10 µg of scrambled siRNA (injection sample No. 3) was injected into a blood vessel in the tail of the mouse. Immediately after injection, by using the pressure applying unit 10a of the delivery device 1, suction was applied at the target site in the liver at 80 kPa for 5 seconds. Thereafter, the abdominal cavity of the mouse was closed, and the mouse was reared for six hours.

Next, the mouse was euthanized under anesthesia. The liver was eviscerated, and the mass of the liver was measured. Thereafter, the liver was placed in the lysis solution in a ratio of 25 mL of the lysis solution per gram of the liver. By using a homogenizer, the resulting mixture was homogenized and dissolved at 4°C to give a pulverized tissue solution. Next, the pulverized tissue solution was centrifuged (10000 x g, for 10 min at 4°C) to collect the supernatant. One-hundred microliters of the luciferin-containing solution was added to 10 µL of the collected supernatant, followed by mixing to give a mixture. Thereafter, the luminometer was used to measure the luciferase activity of the mixture. Thereafter, a relative luciferase activity was calculated, where the luciferase activity obtained when pCMV-Luc was used alone was defined as 100%. A result of an examination in Test Example 3 on a relationship between the type of the injection sample and the relative luciferase activity is shown in FIG. 17.

From the results shown in FIG. 17, the relative luciferase activity obtained when the combination of pCMV-Luc and siRNA-LUC was used was 17% of the relative luciferase activity obtained when pCMV-Luc was used alone. From this result, it can be seen that, in a subject, a nucleic acid injected into a site different from a target site was locally delivered into the target site to which pressure (suction pressure) was applied, and in addition, at the target site, the function of siRNA (RNA interference) was expressed.

### {Test Example 4}

The abdominal cavity of an anesthetized mouse was opened by midline incision to expose a kidney. Next, a pressing unit 22 shown in FIG. 18 was placed in contact with a kidney, and these unit and kidney were sandwiched by two plates to be positionally-fixed. The pressing unit 22 includes a pressing unit body 22a, four balloons 22b integrated to the pressing unit body 22a, and an air supply unit 22d connected to all of the four balloons 22b via a flow path (see FIG. 18).

Next, a syringe was used to inject 100 µg of the pCMV-Luc into a blood vessel in the tail of the mouse. Immediately after injection, the pressing unit 22 was used to press the kidney for 5 seconds at 0 kPa, 30 kPa, 50 kPa, 60 kPa, or 70 kPa. Thereafter, the abdominal cavity of the mouse was closed, and the mouse was reared for six hours.

Next, the mouse was euthanized under anesthesia. The kidney was eviscerated, and the mass of the kidney was measured. Thereafter, by using a homogenizer, the kidney was added to the lysis solution so as to be a ratio of 5 mL of the lysis solution per gram of the kidney. By using a homogenizer, the resulting mixture was homogenized and dissolved at 4°C to give a pulverized tissue solution. Next, the pulverized tissue solution was centrifuged (10000 x g, for 10 min at 4°C) to collect the supernatant. One-hundred microliters of the luciferin-containing solution was added to 10 µL of the collected supernatant, followed by mixing to give a mixture. Thereafter, the luciferase activity of the mixture was measured by using the luminometer. On the other hand, the luciferase activity was measured in the same manner as in the above procedure, except that the kidney was pressed by using a finger in place of using the pressing unit 22. A result of an examination in Test Example 4 on a relationship between the applied air pressure or the method of applying the pressure, and the luciferase activity is shown in FIG. 19.

From the results shown in FIG. 19, the luciferase activity can be measured in the kidney of the mouse. Therefore, from the result, it can be seen that, in a subject, a nucleic acid injected into a site different from a target site was locally delivered into the target site to which pressure (pressing force) was applied, and that a protein encoded by the nucleic acid was expressed at the target site.

From the result shown in FIG. 19, as the magnitude of the applied air pressure increases, the luciferase activity increases. On the other hand, when pressure was applied using a finger, although the luciferase activity can be detected, it was difficult to adjust the expression level thereof. Also, when the pressing unit 22 and the kidney were not sandwiched by the plates, i.e., were not fixed thereon, since only the balloons was caused to make contact with the tissue surface, it was difficult to deliver the nucleic acid. Therefore, this result suggests that the amount of the nucleic acid to be delivered (the expression level) can be set by adjusting the magnitude of the applied air pressure.

### {Test Example 5}

The abdominal cavity of an anesthetized mouse was opened by midline incision to expose a kidney. Next, the kidney S₀ was set in the holding unit body 121 of the holding unit 21 of the delivery device 2 of FIG. 4 (see FIG. 6(A)). Thereafter, the blood vessel side lid portion 122 was attached to the holding unit body 121 (see FIG. 6(B)). In this case, a blood vessel S₂ was allowed to extend out of the holding unit body 121 from a space between the holding unit body 121 and the blood vessel side lid portion 122. Next, the upper lid portion 123 was attached to the holding unit body 121 so that the kidney S₀ was fixed thereon (see FIG. 6(C)). In FIG. 6, the pressing unit 22 is not shown.

Next, the abdominal cavity of the mouse was closed such that only a portion of the pressure applying unit 22 (a portion 22d in FIG. 7) was exposed outside the abdominal cavity of the mouse. By using the supply unit 30 of the delivery device 2, 100 µg of the pCMV-Luc was injected into a blood vessel in the tail of the mouse. Immediately after injection, by using the pressing unit 22 of the delivery device 2, the kidney was pressed at 60 kPa (applied air pressure) for 5 seconds. Thereafter, the mouse was reared for six hours.

Next, the mouse was euthanized under anesthesia. After the kidney was eviscerated, the mass of the kidney was measured. Thereafter, by using a homogenizer, the kidney was added to the lysis solution in a ratio of 5 mL of the lysis solution per gram of the kidney. By using a homogenizer, the kidney in the resulting mixture was homogenized and dissolved at 4°C to give a pulverized tissue solution. Next, the pulverized tissue solution was centrifuged (10000 x g, for 10 min at 4°C) to collect the supernatant. One-hundred microliters of the luciferin-containing solution was added to 10 µL of the collected supernatant, to give a mixture. Thereafter, the luciferase activity of the mixture was measured by using the luminometer. On the other hand, the luciferase activity was measured in the same manner as in the above procedure, except that the applied air pressure was set to 0 kPa in place of 60 kPa. A result of an examination in Test Example 5 on a relationship between the pressure application conditions and the luciferase activity is shown in FIG. 20.

From the results shown in FIG. 20, when the nucleic acid is injected into a blood vessel in the tail of a mouse and thereafter the kidney is pressed while being positionally-fixed by the holding unit 21, the luciferase activity can be measured in the kidney of the mouse. Therefore, from this result, it can be seen that the nucleic acid is locally delivered into the target site, and that a protein encoded by the nucleic acid is expressed at the target site, when a nucleic acid is injected into a site different from a target site in a subject, and thereafter the target site is pressed while being fixed by the holding unit.

Also, from the results shown in FIG. 20, when the delivery device 2 having the holding unit 21 (see FIG. 4) is used, it can be seen that a nucleic acid can be locally delivered into a target site even after the abdominal cavity is closed. In contrast, when an organ having a target site is fixed by two plates (see Test Example 4), it is difficult to close the abdominal cavity. Therefore, it is considered that a nucleic acid can be delivered into a target site only when the abdominal cavity is open. Therefore, these results suggest that when the delivery device 2 having the holding unit is used, a nucleic acid can be delivered into a target site many times without repeatedly opening the abdominal cavity.

From the above result, it can be seen that the substance to be introduced such as a nucleic acid or the like can be locally delivered into the target site, and that a function of the substance to be introduced such as a nucleic acid or the like (for example, when the substance to be introduced is a nucleic acid, the expression of a protein encoded by the nucleic acid, a function of the nucleic acid, and the like) can be expressed at the target site, when a substance to be introduced such as a nucleic acid or the like is injected into a site different from a target site in a subject and thereafter pressure (suction pressure or pressing force) is applied to the target site. Also, it can be seen that the substance to be introduced such as a nucleic acid or the like can be efficiently delivered into the target site by positionally-fixing the target site with the holding unit, when pressing force is applied to the target site. Therefore, it can be seen that the present invention is useful for locally delivering a nucleic acid drug or the like into a target site.

### {Example 5}

The luciferase activity was measured in the same manner as in Example 4, except that the kidney, heart, spleen, or liver was used as the subject, and that conditions for the pressure applying unit 10a placed at a target site in the subject were those shown in Table 3. The results of an examination in Example 5 on a relationship between the type of the subject and the luciferase activity are shown in FIG. 21.

**{Table 3}**

| | Inner diameter D₁(mm) | Outer diameter D₂(mm) | Height h(mm) | Suction scheme |
|---|---|---|---|---|
| Kidney | 2 | 3 | 3 | Only one site |
| Heart | 2 | 3 | 3 | Only one site |
| Spleen | 2 | 3 | 3 | Only one site |
| Liver | 2 | 3 | 3 | Only one site |

From the results shown in FIG. 21, the luciferase activity is detected in all of the kidney, heart, spleen, and liver. Therefore, from these results, it can be seen that, in all of the kidney, heart, spleen, and liver, a nucleic acid injected into a site different from a target site was locally delivered into the target site to which pressure (suction pressure) was applied, and that a protein encoded by the nucleic acid was expressed at the target site.

### {Example 6}

The luciferase activity was measured in the same manner as in Example 4, except that the heart was used as a subject in place of the kidney, and that delivery devices (device Nos.: 5 to 7) shown in Table 4 were used as the delivery device 1. A result of an examination in Example 6 on a relationship between the type of the device used and the luciferase activity is shown in FIG. 22.

**{Table 4}**

| Device number | Inner diameter D₁(mm) | Outer diameter D₂(mm) | Height h(mm) | Suction scheme |
|---|---|---|---|---|
| 5 | 2 | 3 | 3 | Only one site |
| 6 | 2 | 3 | 3 | Only two sites |
| 7 | 3 | 4 | 3 | Only one site |

From the result shown in FIG. 22, it can be seen that the luciferase activity obtained in the case where the number of suction pressure-applied sites was two was higher than the luciferase activity obtained in the case where the number of suction pressure-applied sites was one, when the suction part having the same size was used. Therefore, this result suggests that the amount of a nucleic acid to be delivered or the place to which a nucleic acid is to be delivered can be set by repeatedly performing suction,.

Also, the luciferase activity obtained in the case of using a suction part having an inner diameter D₁ of 3 mm and an outer diameter D₂ of 4 mm was higher than the luciferase activity obtained in the case of using a suction part having an inner diameter D₁ of 2 mm and an outer diameter D₂ of 3 mm, when the number of sites to which suction pressure was applied was the same. Therefore, this result suggests that the amount of a nucleic acid to be delivered can be set by adjusting the size of the suction part.

### {Test Example 6}

The abdominal cavity of an anesthetized mouse was opened by midline incision to expose the kidney. Thereafter, the pressure applying unit 10a of the delivery device 1 (see FIG. 2 and FIG. 3) was placed at a target site in the kidney. As the delivery device 1, a device shown in Table 5 was used.

**{Table 5}**

| Device number | Inner diameter D₁(mm) | Outer diameter D₂(mm) | Height h(mm) | Suction scheme |
|---|---|---|---|---|
| 8 | 3 | 5 | 3 | Only one site |

Bovine serum albumin (BSA) was added to phosphate buffer solution (PBS) so as to have a concentration of 0.1 mass%, to give 0.1 mass% BSA-PBS solution. Firefly luciferase enzyme solution (manufactured by TOYO B-net, CO., LTD.) was added to the resulting 0.1 mass% BSA-PBS solution so as to have a firefly luciferase concentration of 0.04 µg/200 µL, to give a mixture.

Next, the mixture was injected into a blood vessel in the tail of a mouse, by using the supply unit 30 of the delivery device 1. Immediately after injection, by using the pressure applying unit 10a of the delivery device 1, suction pressure was applied to a target site in one of the kidneys for 5 seconds under conditions shown in Table 5. Immediately after applying suction pressure, the luciferase activity was measured.

Next, after the mouse was euthanized by bloodletting under anesthesia, both the kidneys were eviscerated. The masses of the kidneys were measured.

Thereafter, the kidney having the target site to which the suction pressure was applied was added to a lysis solution so as to be a ratio of 10 mL of the lysis solution per gram of the kidney. By using a homogenizer, the resulting mixture was homogenized and dissolved at 4°C to give a pulverized tissue solution. Next, the pulverized tissue solution was centrifuged (10000 x g, for 10 min at 4°C) to collect the supernatant. One-hundred microliters of the luciferin-containing solution was added to 10 µL of the collected supernatant, followed by mixing to give a mixture. Thereafter, the luciferase activity of the mixture was measured by using a luminometer (trade name: Lumat LB 9507, manufactured by EG&G BERTHOLD) (an introduction method of experiment No. 1).

On the other hand, the luciferase activity was measured in the same manner as in the above, except that the kidney to which suction pressure was not applied in place of the kidney having a target site to which suction pressure was applied (an introduction method of experiment No. 2).

Next, a relative activity was calculated, where the luciferase activity obtained when the method of experiment No. 2 was performed was defined as 100%. A result of an examination in Test Example 6 on a relationship between the type of the introduction method and the relative luciferase activity is shown in FIG. 23.

From the result of FIG. 23, it can be seen tha the luciferase activity obtained in the case where the introduction method of experiment No. 1 was performed was higher than the luciferase activity obtained in the case where the introduction method of experiment No. 2 was performed. Therefore, from this result, it can be seen that the protein injected to a site different from the target site was locally delivered into the target site to which pressure (suction pressure) was applied, and that the physiological activity of the protein was expressed at the target site.

From the above result, by injecting a substance to be introduced such as a nucleic acid, a peptide compound, or the like into a site different from a target site in a subject and thereafter applying pressure (suction pressure or pressing force) to the target site, it can be seen that, the substance to be introduced can be locally delivered into the target site and that a function of the substance to be introduced can be expressed at the target site. Therefore, the present invention is useful for locally delivering various substances to be introduced such as a nucleic acid, a pharmaceutical, a peptide compound, an antibody, a high molecular compound, a metal (for example, a metal nanoparticle, and the like), a semiconductor (for example, a quantum dot, and the like), a substance composed of a combination thereof, and the like, into a target site in therapy or prevention of human diseases, local delivery of these substances to be introduced into a target site in diagnosis, and the like.

### {Test Example 7}

After a mixture obtained by adding to a solvent a pharmaceutical, an antibody, a high molecular compound, a metal nanoparticle, or a quantum dot is injected into a site different from a target site in a subject, suction pressure or pressing force is applied to the target site. As a result, the pharmaceutical, antibody, high molecular compound, metal nanoparticle, or quantum dot can be locally delivered into the target site. By using a detection method in accordance with each substance, the presence of the pharmaceutical, antibody, high molecular compound, metal nanoparticle or quantum dot in the target site can be confirmed.

### DESCRIPTION OF THE REFERENCE CHARACTERS

- 1: delivery device
- 2: delivery device
- 10: pressure applying unit
- 10a: pressure applying unit
- 10b: pressure applying unit
- 11: suction part
- 11a: suction opening
- 12: connection part
- 13: pipe member
- 21: holding unit
- 22: pressing unit
- 22a: pressing unit body
- 22b: balloon
- 22c: flow path
- 22d: fluid inlet
- 23: port
- 24: flow path unit
- 25: fluid supply unit
- 25a: holding unit
- 26: fluid reservoir unit
- 27: converter
- 29: needle unit
- 30: supply unit
- 30a: reservoir part
- 30b: discharge part
- 40: control unit
- 121: holding unit body
- 121a: bottom portion
- 121b: lateral portion
- 121c: lateral portion
- 121d: lateral portion
- 122: vascular path side lid portion
- 123: upper lid portion
- 131: protruding portion
- 132: end portion
- 133: groove portion
- 134: stopper
- 135: groove portion
- S₀: tissue
- S₁: target site
- S₂: vascular path (blood vessel)

## Claims

1. A method for operating a device for delivering a substance to be introduced, the device comprising
a supply unit configured to supply the substance to be introduced to a vascular path located upstream of a target site in a tissue existed in a living organism, and
a pressure applying unit configured to apply pressure to the target site in the tissue,
the method comprising the steps of:
(1-1) causing the supply unit to discharge the substance to be introduced, thereby supplying the substance to be introduced to the vascular path and
(1-2) simultaneously with or after the step (1-1), causing the pressure applying unit to generate pressure against the target site in the tissue.

2. The method according to claim 1, wherein the substance to be introduced is a nucleic acid or a peptide compound.

3. The method according to claim 1 or 2, wherein the pressure applying unit comprises a suction part configured to apply suction at the target site, and
wherein in the step (1-2), suction pressure against the target site is generated by causing the suction part to apply suction at the target site.

4. The method according to claim 1 or 2, wherein the pressure applying unit comprises a pressing unit configured to press the target site, and a holding unit configured to positionally-fix and hold the tissue, and
wherein in the step (1-2), a pressing force against the target site is generated by causing the pressing unit to press the target site.

5. The method according to claim 4, wherein the holding unit is implantable in the living organism.

6. A method for delivering a substance to be introduced into a target site in a tissue of a non-human mammal, the method comprising the steps of:
(2-1) supplying the substance to be introduced to a vascular path located upstream of the tissue; and
(2-2) simultaneously with or after the step (2-1), applying pressure to the target site in the tissue, thereby delivering the substance to be introduced into the target site.

7. The method according to claim 6, wherein the substance to be introduced is a nucleic acid or a peptide compound.

8. The method according to claim 6 or 7, wherein in the step (2-2), suction pressure is applied to the target site.

9. The method according to claim 6 or 7, wherein in the step (2-2), the target site is pressed while positionally-fixing the tissue.

10. The method of claim 9, wherein the tissue is a tissue of an organ, and wherein in the step (2-2), the target site is pressed while the tissue is positionally-fixed, the organ being accommodated in a case capable of accommodating the organ.

11. A method for delivering a substance to be introduced into a target site in a subject derived from a living organism, the method comprising the steps of:
(3-1) supplying the substance to be introduced to a site different from the target site in the subject; and
(3-2) simultaneously with or after the step (3-1), applying pressure to the target site in the subject, thereby delivering the substance to be introduced into the target site.

12. The method according to claim 11, wherein the substance to be introduced is a nucleic acid or a peptide compound.

13. The method according to claim 11 or 12, wherein in the step (3-2), suction pressure is applied to the target site.

14. The method according to claim 11 or 12, wherein in the step (3-2), the target site is pressed while positionally-fixing the target site.
